(19) Europäisches Patentamt / European Patent Office / Office européen des brevets

(11) **EP 3 583 250 B1**

(12) **EUROPEAN PATENT SPECIFICATION**

(45) Date of publication and mention of the grant of the patent:
**12.07.2023 Bulletin 2023/28**

(21) Application number: **17896462.3**

(22) Date of filing: **15.12.2017**

(51) International Patent Classification (IPC):
*G16B 50/50* (2019.01)    *G16B 30/20* (2019.01)
*G06F 21/62* (2013.01)    *H03M 7/30* (2006.01)

(52) Cooperative Patent Classification (CPC):
**H03M 7/3086; G06F 21/6245; G16B 30/20; G16B 50/50; H03M 7/3088; H03M 7/3091; H03M 7/3095; H03M 7/70**

(86) International application number:
**PCT/US2017/066863**

(87) International publication number:
**WO 2018/151788 (23.08.2018 Gazette 2018/34)**

(54) **METHOD AND SYSTEMS FOR THE EFFICIENT COMPRESSION OF GENOMIC SEQUENCE READS**

VERFAHREN UND SYSTEME ZUR EFFIZIENTEN KOMPRIMIERUNG GENOMISCHER SEQUENZAUSLESUNGEN

PROCÉDÉ ET SYSTÈMES POUR LA COMPRESSION EFFICACE DE LECTURES DE SÉQUENCE GÉNOMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2017 PCT/US2017/017842**
**11.07.2017 PCT/US2017/041579**

(43) Date of publication of application:
**25.12.2019 Bulletin 2019/52**

(73) Proprietor: **Genomsys SA**
**1015 Lausanne (CH)**

(72) Inventors:
• **BALUCH, Mohamed, Khoso**
**Chantilly, VA 20151 (US)**
• **ALBERTI, Claudio**
**1213 Petit-Lancy (Geneva) (CH)**

(74) Representative: **Metroconsult Srl**
**Foro Bonaparte 51**
**20121 Milano (IT)**

(56) References cited:
**US-A1- 2015 227 686    US-A1- 2016 100 177**

• Anonymous: "CRAM format specification (version 3.0)", , 8 September 2016 (2016-09-08), XP002771305, Retrieved from the Internet: URL:https://samtools.github.io/hts-specs/CRAMv3.pdf [retrieved on 2017-06-22]
• PARIDAENS et al.: "AFRESh: an adaptive framework for compression of reads and assembled sequences with random access functionality", Bioinformatics, vol. 33, no. 10, 5 January 2017 (2017-01-05), pages 1464-1472, XP055537212,
• PENG et al.: "Re-alignment of the unmapped reads with base quality score", BMC Bioinformatics, vol. 16, no. Suppl 5, 18 March 2015 (2015-03-18), page S8, XP021217805,
• "Java CIGAR Parser for SAM format", Bioinformatics I/O, 16 November 2015 (2015-11-16), XP055537219, Retrieved from the Internet: URL:http://bioinformatics.cvr.ac.uk/blog/tag/cigar-string [retrieved on 2018-06-19]
• DAILY et al.: "Data structures and compression algorithms for high-throughput sequencing technologies", BMC Bioinformatics, vol. 11, 14 October 2010 (2010-10-14), page 514, XP021071841,

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 583 250 B1

- **PANNEEL: "Implementation and optimization of CABAC in a genomic data compression framework with support for random access", Master's dissertation, Faculty of Engineering and Architecture,, August 2015 (2015-08), XP055535469,**

## Description

### TECHNICAL FIELD

This disclosure provides a novel method of representation of genome sequencing data which reduces the utilized storage space and improves access performance by providing new functionality that are not available with known prior art methods of representation.

### BACKGROUND

An appropriate representation of genome sequencing data is fundamental to enable efficient genomic analysis applications such as genome variants calling and all other analysis performed with various purposes by processing the sequencing data and metadata.

Human genome sequencing has become affordable by the emergence of high-throughput low cost sequencing technologies. Such opportunity opens new perspectives in several fields ranging from the diagnosis and treatment of cancer to the identification of genetic illnesses, from pathogen surveillance for the identification of antibodies to the creation of new vaccines, drugs and the customization of personalized treatments.

Hospitals, genomic analysis providers, bioinformatics and large biological data storage centers are looking for affordable, fast, reliable and interconnected genomic information processing solutions which could enable scaling genomic medicine to a world-wide scale. Since one of the bottleneck in the sequencing process has become data storage, methods for representing genome sequencing data in a compressed form are increasingly investigated.

The most used genome information representations of sequencing data are based on zipping FASTQ and SAM formats. The objective is to compress the traditionally used file formats (respectively FASTQ and SAM for non-aligned and aligned data). Such files are constituted by plain text characters and are compressed, as mentioned above, by using general purpose approaches such as LZ (from Lempel and Ziv, the authors who published the first versions) schemes (the well-known zip, gzip etc). When general purpose compressors such as gzip are used, the result of compression is usually a single blob of binary data. The information in such monolithic form results quite difficult to archive, transfer and elaborate particularly when like in the case of high throughput sequencing the volume of data are extremely large. The BAM format is characterized by poor compression performance due to the focus on compression of the inefficient and redundant SAM format rather than on extracting the actual genomic information conveyed by SAM files and due to the adoption of general purpose text compression algorithms such as gzip rather than exploiting the specific nature of each data source (the genomic data itself).

A more sophisticated approach to genomic data compression that is less used, but more efficient than SAM is CRAM.

CRAM technique is disclosed, for example, in internet publication "CRAM format specification (version 3.0)", dated 8 September, 2016 - https://samtools.github.io/hts-specs/CRAMv3.pdf.

CRAM provides more efficient compression for the adoption of differential encoding with respect to a reference (it partially exploits the data source redundancy), but it still lacks features such as incremental updates, support for streaming and selective access to specific classes of compressed data.

These approaches generate poor compression ratios and data structures that are difficult to navigate and manipulate once compressed. Downstream analysis can be very slow due to the necessity of handling large and rigid data structures even to perform simple operation or to access selected regions of the genomic dataset. CRAM relies on the concept of the CRAM record. Each CRAM record represents a single mapped or unmapped reads by coding all the elements necessary to reconstruct it.

CRAM presents the following drawbacks and limitations that are solved and removed by the invention described in this disclosure:

> 1. CRAM does not support data indexing and random access to data subsets sharing specific features. Data indexing is out of the scope of the specification (see section 12 of CRAM specification v 3.0) and it is implemented as a separate file. Conversely the approach of the invention described in this disclosure employs a data indexing method that is integrated with the encoding process and indexes are embedded in the encoded (i.e. compressed) bit stream.
> 2. CRAM is built by core data blocks that can contain any type of mapped reads (perfectly matching reads, reads with substitutions only, reads with insertions or deletions (also referred to as "indels")). There is no notion of data classification and grouping of reads in classes according to the result of mapping with respect to a reference sequence. This means that all data need to be inspected even if only reads with specific features are searched. Such limitation is solved by the invention by classifying and partitioning data in classes before coding.
> 3. CRAM is based on the concept of encapsulating each read into a "CRAM record". This implies the need to inspect each complete "record" when reads characterized by specific biological features (e.g. reads with substitutions, but without "indels", or perfectly mapped reads) are searched.

Conversely, in the present invention there is the notion of data classes coded separately in separated information blocks and there is no notion of record encapsulating each read. This enables more efficient access to set of reads with specific biological characteristics (e.g. reads with substitutions, but without "indels", or perfectly mapped reads) without the need of decoding each (block of) read(s) to inspect its features.

4. In a CRAM record each record field is associated to a specific flag and each flag must always have the same meaning as there is no notion of context since each CRAM record can contain any different type of data. This coding mechanism introduces redundant information and prevents the usage of efficient context based entropy coding.

Instead in the present invention, there is no notion of flag denoting data because this is intrinsically defined by the information "block" the data belongs to. This implies a largely reduced number of symbols to be used and a consequent reduction of the information source entropy which results into a more efficient compression. Such improvement is possible because the use of different "blocks" enables the encoder to reuse the same symbol across each block with different meanings according to the context. In CRAM each flag must always have the same meaning as there is no notion of contexts and each CRAM record can contain any type of data.

5. In CRAM substitutions, insertions and deletions are represented by using different syntax elements, option that increases the size of the information source alphabet and yields a higher source entropy. Conversely, the approach of the disclosed invention uses a single alphabet and encoding for substitutions, insertions and deletions. This makes the encoding and decoding process simpler and produces a lower entropy source model which coding yields bitstreams characterized by high compression performance.

The present invention aims at compressing genomic sequences by classifying and partitioning sequencing data so that the redundant information to be coded is minimized and features such as selective access and support for incremental updates are directly enabled in the compressed domain.

One of the aspects of the presented approach is the definition of classes of data and metadata structured in different blocks and encoded separately. The more relevant improvements of such approach with respect to existing methods consist in:

1. the increase of compression performance due to the reduction of the information source entropy constituted by providing an efficient source model for each class of data or metadata;

2. the possibility of performing selective accesses to portions of the compressed data and metadata for any further processing purpose directly in the compressed domain;

3. the possibility to incrementally (i.e. without the need of decoding and re-encoding) update compressed data and metadata with new sequencing data and/or metadata and/or new analysis results associated to specific sets of sequencing reads.

## BRIEF DESCRIPTION OF THE DRAWINGS

Figure 1 shows the definition of read 1 and read 2 in a read pair and the definition of left-most and right-most nucleotide in a mapped read.

Figure 2 shows how an Access Unit encapsulates compressed descriptors representing sequence reads mapped in a contiguous interval of the reference sequence. Header information is prepended to the compressed descriptors in order to enable data parsing.

Figure 3 shows how an Access Unit of type P is composed by a header and the multiplexing of blocks of descriptors representing the reads mapping positions (pos), the reverse complement information (rcomp), the pairing information in case of paired end reads (pair), the reads length in case of variable reads length (rlen) and mapping flags (flags). It is used to encode reads of class P.

Figure 4 shows the calculation of the pos descriptor for a mapped read pair where read pair $n$ is mapped at position $P_n$ and read pair n+1 is mapped at position $P_m$.

Figure 5 shows how to How to calculate the absolute mapping position of one base on a Reference Sequence.

Figure 6 shows the use of the rcomp descriptor for paired end reads.

Figure 7 shows an example of how to calculate mismatches positions in a read pair.

Figure 8 shows how the Genomic Record Lenght and the Pairing Distance are calculated.

Figure 9 shows multiple alignments without splicing. The left-most read has N alignments. N is the first value of mmap to be decoded and signals the number of alignments of the first read. The following N values of the mmap descriptor are decoded and are used to calculate P which is the number of alignments of the second read.

Figure 10 shows how the pos, pair and mmap descriptors are used to encode multiple alignments without splices.

The left-most read has N alignments.

Figure 11 shows multiple alignments with splices.

Figure 12 shows multiple alignments with splices. Use of the pos, pair, mmap and msar descriptors to represent multiple alignments with splices.

Figure 13 depicts an encoder apparatus comprising the steps of aligning genomic sequences with respect to a reference genome, generating descriptors representing the genomic sequences with respect to the reference genome, compressing each block of descriptors with a dedicated entropy coder.

Figure 14 shows the decoding process of a compressed bitstream comprising the steps of demultiplexing the incoming bitstream to extract the entropy coded descriptors, entropy decoding of each type of descriptors, decoding of aligned sequence reads using the reference genome.

Figure 15 shows how encoders of data of class N, M and I are configured with vectors of thresholds and generate separate subclasses of N, M and I data classes.

Figure 16 shows how half mapped read pairs (class HM) can be used to fill unknown regions of a reference sequence by assembling longer contigs with unmapped reads.

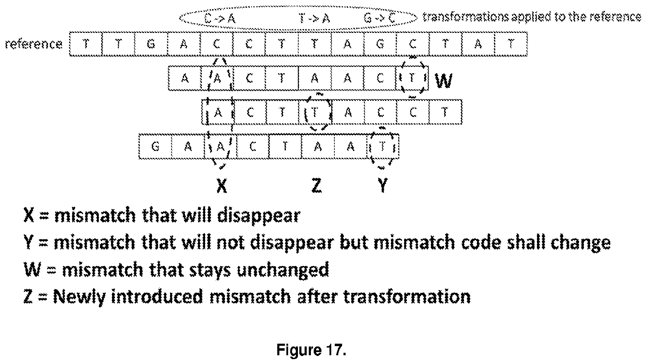

Figure 17 shows how reference transformations can be applied to remove mismatches from reads. In some cases reference transformations may generate new mismatches or change the type of mismatches found when referring to the reference before the transformation has been applied.

Figure 18 shows how reference transformations can change the class reads belong to when all or a subset of mismatches are removed (i.e. the read belonging to class M before transformation is assigned to Class P after the transformation of the reference has been applied).

Figure 19 shows how all classes of data can use the same transformed reference for re-encoding or a different transformation can be used for each class N, M and I, or any combination thereof.

## SUMMARY

In a first aspect, the invention refers to a computer-implemented method for encoding genome sequence data according to claim 1.

In a second aspect, the invention refers to a computer-implemented method for decoding genome sequence data according to claim 11.

In a third aspect, the invention refers to an apparatus for encoding genome sequence data according to claim 17.

In a fourth aspect, the invention refers to an apparatus for decoding genome sequence data according to claim 18.

In a fifth aspect, the invention refers to storage means comprising instructions for executing encoding and decoding methods according to claim 19.

## DETAILED DESCRIPTION

The genomic or proteomic sequences referred to in this invention include, for example, and not as a limitation, nucleotide sequences, Deoxyribonucleic acid (DNA) sequences, Ribonucleic acid (RNA), and amino acid sequences. Although the description herein is in considerable detail with respect to genomic information in the form of a nucleotide sequence, it will be understood that the methods and systems for compression can be implemented for other genomic or proteomic sequences as well, albeit with a few variations, as will be understood by a person skilled in the art.

Genome sequencing information is generated by High Throughput Sequencing (HTS) machines in the form of sequences of nucleotides (a. k. a. "bases") represented by strings of letters from a defined vocabulary. The smallest vocabulary is represented by five symbols: {A, C, G, T, N} representing the 4 types of nucleotides present in DNA namely Adenine, Cytosine, Guanine, and Thymine. In RNA Thymine is replaced by Uracil (U). N indicates that the sequencing machine was not able to call any base and so the real nature of the position is undetermined. In case the IUPAC ambiguity codes are adopted by the sequencing machine, the alphabet used for the symbols is (A, C, G, T, U, W, S, M, K, R, Y, B, D, H, V, N or -).

The nucleotides sequences produced by sequencing machines are called **"reads"**. Sequence reads can be between a few dozens to several thousand nucleotides long. Some technologies produce sequence reads in **"pairs"** where one read is from one DNA strand and the second is from the other strand. A read associated to another read in a sequencing process producing pairs is said to be its **mate.**

The process of arranging sequence reads to identify regions of similarity with segments of a reference genome according to a set of matching rules is called "alignment" or "mapping". Throughout this disclosure, a **reference sequence** is a sequence of nucleotides associated to a mono-dimensional integer coordinate system for which each integer coordinate is associated to a single nucleotide. Coordinate values can only be equal or larger than zero. This coordinate system in the context of this invention is zero-based (i.e. the first nucleotide has coordinate 0 and it is said to be at position 0) and linearly increasing from left to right. A **reference sequence** is any sequence on which the nucleotides sequences produced by sequencing machines are aligned/mapped. One example of sequence could actually be a **"reference genome"**, a sequence assembled by scientists as a representative example of a species' set of genes. For example GRCh37, the Genome Reference Consortium human genome (build 37) is derived from thirteen anonymous volunteers from Buffalo, New York. However, a reference sequence could also consist of a synthetic sequence conceived and constructed to merely improve the compressibility of the reads in view of their further processing.

A common element of efficient approaches to genomic sequence reads compression is the exploitation of the correlation of sequence data with respect to a reference sequence. Even if the somatic profile of the human population is extremely diversified, the actual portion of the number of nucleotides that differs from person to person is about only 0.1% of the total number of nucleotides composing an entire genome. Therefore, the specific genomic information characterizing each individual is very limited with respect to the entire information carried by an entire genome. When a pre-existing reference genome is available, be it for previous sequencing or as a published "average" consensus reference, the most common way as of today to encode the information is to identify and encode only the differences with respect to the reference genome.

In order to do so with raw sequence reads, generally expressed in the form of FASTQ data files, a preliminary pre-processing step the mapping on a reference genome. In case an appropriate reference genome is not available, or if the bias introduced by the usage of a specific reference is not desirable, the construction of a new reference sequence by means of assembling the sequence reads at hand into longer sequences called **contigs,** is a possible alternative.

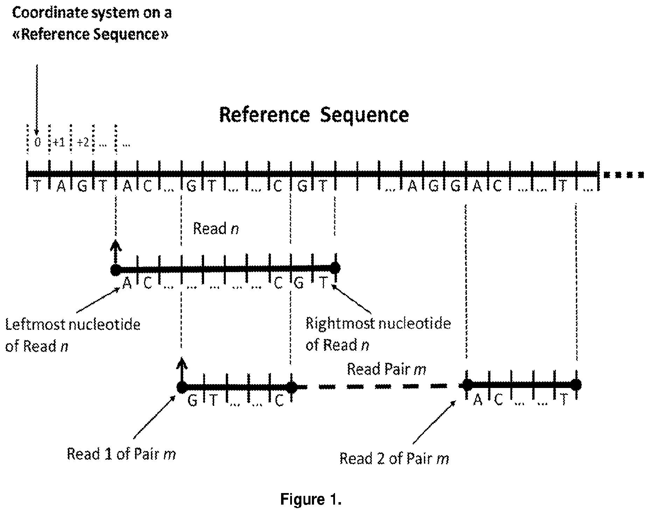

When mapping sequence reads on a reference sequence, said reference sequence is used as axis of a mono-dimensional coordinate system in which the left-most position is denoted as position 0. For each sequence read, mapped to a reference sequence, the nucleotide mapped at the reference sequence position identified by the smallest coordinate number is usually referred to as the "left-most" nucleotide, whereas the nucleotide mapped at the reference sequence position identified by the largest coordinate number is referred to as the "right-most" nucleotide. This is illustrated in Figure 1. Throughout this disclosure, a nucleotide is also referred to as a **base.**

When a sequence read is mapped to a reference sequence, the coordinate of the left-most mapped base is said to represent the **mapping position** of the read on the reference sequence.

A base present in the aligned read and not present in the reference sequence (a.k.a. insertion) and bases preserved by the alignment process but not mapped on the reference sequence (a.k.a. soft clips) do not have mapping positions.

When a sequence read cannot be mapped to any mapped position of the used reference sequences according to the specified matching rules, it is said to be **unmapped.**

The process of building longer genomic sequences by looking for overlapping regions among sequence reads is called **assembly.**

A longer genomic sequence built assembling shorter reads is called **contig** (see https://en.wikipedia.org/wiki/Contig).

Sequence reads that fail to build any contig during an assembly process are said to be **unaligned.**

A **reference genome** is composed by one or more reference sequences and it is assembled by scientists as a representative example of a species' set of genes. For example GRCh37, the Genome Reference Consortium human genome (build 37) is derived from thirteen anonymous volunteers from Buffalo, New York. However, a reference sequence could also consist of a synthetic sequence conceived and merely constructed to improve the compressibility of the reads in view of their further processing.

In this disclosure the read composing a read pair with a base mapping on the smallest coordinate on a reference sequence is referred to as **"Read 1"** whereas its mate is referred to as **"Read 2".**

The distance, expressed as number of nucleotides (or bases), separating two reads generated as a pair, by a sequencing machine using current state of the art sequencing technology, is unknown, and it is determined by mapping both reads composing the pair (i.e. minimizing appropriate matching functions) to a reference sequence.

Throughout this disclosure, a **genomic record** is a data structure encoding either a single sequence read or a paired sequence read optionally associated with alignment information, read identifier and quality values.

Throughout this disclosure, an **Access Unit** (AU) is defined as a logical data structure containing a coded representation of genomic information or related metadata to facilitate the bit stream access and manipulation. It is the smallest data organization that can be decoded by a decoding device implementing the invention described in this disclosure.

According to the type of coded information, an AU can be decoded either independently of any other AU or using information contained in other AUs.

AUs can be classified into a multiplicity of types according to the nature of the coded sequence data. An Access Unit contains either a reference sequence, or a portion thereof, or encoded reads or read pairs belonging to a single class of data. Any single AU cannot contain two or more types of sequence data. For example an Access Unit may contain the entire chromosome 1 of GRCh37, the Genome Reference Consortium human genome (build 37). Another Access Unit may contain the coded representation of nucleotides of chromosome 1 of GRCh37 that are located between coordinates 50'000 and 150'000. Another Access Unit may contain only reads or read pairs that perfectly map on the reference sequence without any mismatch. Another Access Unit may contain reads or read pairs that only contain "N" symbols as mismatches with respect to the reference sequence. Another Access Unit may contain reads or read pairs that contain any type of substitutions (e.g. one base present in the read or read pair is different from the base at the corresponding mapping position in the reference sequence). Another Access Unit may contain reads or read pairs that contain mismatches, insertions, deletions and soft clipped bases. Another Access Unit may contain only read or read pairs that do not map on the reference sequence. Another Access Unit may contain only read pairs in which one read is mapped and the other is not mapped on the reference sequence. Another type of Access Unit may contain only encoded segments of a reference genome composed by one or more reference sequences (for example chromosomes).

The essential characteristic of an Access Unit is that it contains in compressed form all elements needed to reconstruct the genomic information (sequence reads or read pairs, reference sequences), associated alignment information and metadata of reads or read pairs it represents. In other words, to fully reconstruct the reads, read pairs or reference sequence and associated information carried by an Access Unit it is only necessary to retrieve the Access Unit itself and, when applicable, the Access Units containing the reference sequence it refers to.

In each Access Unit the descriptors listed in the next section representing the encoded read or read pairs are aggregated in separate data blocks - one per type - in order to exploit their homogeneous statistical properties for achieving high performance entropy coding.

Each Access Unit contains the compressed sub-set of descriptors representing sequence reads or read pairs belonging to the same class mapped to a genomic region on a reference sequence. Such genomic region on the reference sequence is defined by a start coordinate (or start position) and an end coordinate (or end position).

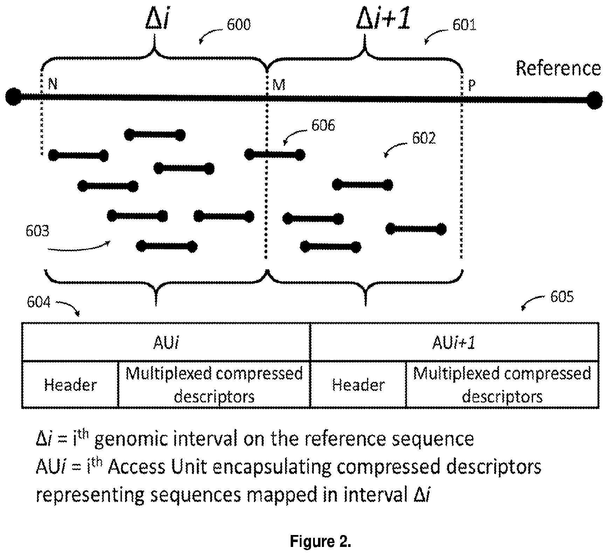

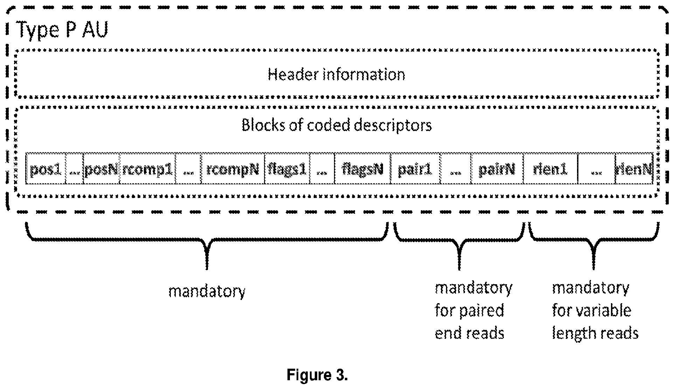

An example of Access Unit is illustrated in figure 2 and figure 3. Access Units are composed by blocks of encoded genomic descriptors (described in the next section). To enable transport over a network, blocks are further decomposed into packets. When compressing genomic sequence reads, each Access Units contains compressed descriptor representing either sequence reads mapped to a genomic interval on the reference sequence or unmapped sequence reads. Access Units can be used to carry reference genomes or parts thereof. A reference sequence can be either encoded as a single long sequence of nucleotides or split into shorter sequences encoded as unmapped genomic sequence reads.

In the context of this disclosure the following definitions apply:

> **access unit start position:** left-most genomic record position among all genomic records contained in the access unit.
> **access unit end position:** right-most base position among all mapped bases of all genomic records contained in the access unit.
> **access unit range:** the genomic range comprised between the access unit start position and the right-most genomic record position among all genomic records contained in the access unit. **access unit size:** number of genomic records contained in an access unit.
> **access unit covered region:** genomic range comprised between the Access Unit start position and the Access Unit end position.

In the context of this disclosure, one or more access unit are organized in a structure called **genomic dataset.** A **genomic dataset** is a compression unit containing headers and access units. The set of access units composing the **genomic dataset** constitutes the **genomic dataset payload.**

A collection of one or more **genomic datasets** is called **dataset group.**

In the context of this disclosure, **genomic descriptors** are syntax elements representing part of the information (and also elements of a syntax structure of a file format and/or a bitstream) necessary to reconstruct (i.e. decode) coded reference sequences, sequence reads and the associated mapping information. The genomic descriptors disclosed in this invention are listed in Table 3.

According to the method disclosed in this invention, reference sequences or portion thereof, sequence reads and the associated alignment information are coded using a sub-set of the descriptors listed above which are then entropy coded using a multiplicity of entropy coders according to each descriptor specific statistical properties. Blocks of compressed descriptors with homogeneous statistical properties are structured in Access Units which represent the smallest coded representation of one or more genomic sequence that can be manipulated by a device implementing the invention described in this disclosure.

Genomic descriptors are organized in **blocks** and **streams** as defined below.

A **block** is defined as a data unit composed by a header and a payload, which is composed by portions of compressed descriptors of the same type.

A **descriptor stream** is defined as a sequence of encoded descriptor blocks used to decode a descriptor of a specific Data Class.

Sequencing devices can introduce errors in the sequence reads such as:

1. the decision of skipping a base call due to the lack of confidence in calling any specific base. This is called an unknown base and labeled as "N" (denoted as mismatch of "n type");
2. the use of a wrong symbol (i.e. representing a different nucleic acid) to represent the nucleic acid actually present in the sequenced sample; this is usually called "substitution error" (denoted as mismatch of "s type");
3. the insertion in one sequence read of additional symbols that do not refer to any actually present nucleic acid; this is usually called "insertion error" (denoted as mismatch of "i type");
4. the deletion from one sequence read of symbols that represent nucleic acids that are actually present in the sequenced sample; this is usually called "deletion error" (denoted as mismatch of "d type");
5. the recombination of one or more fragments into a single fragment which does not reflect the reality of the originating sequence; this usually results in aligners decision to clip bases (denoted as mismatch of "c type").

In genome sequencing the term **"coverage"** is used to express the level of redundancy of the sequence data with respect to a **"reference sequence"**. The average coverage of aligned genome sequencing data is the average number of time each base at each position of the reference genome is present in the aligned data. For example, to reach a coverage of 30x on a human genome (3.2 billion bases long) a sequencing machine shall produce a total of 30 x 3.2 billion bases so that in average each position in the reference is "covered" 30 times.

The coverage is said to be:

- partial (less than 1X) when some parts of the reference genome are not mapped by any available sequence read;
- single (1X) when all nucleotides of the reference genome are mapped by one and only one symbol present in the sequence reads;
- multiple (2X, 3X, NX) when each nucleotide of the reference genome is mapped multiple times.

This invention aims at defining a genomic information representation format in which the relevant information is efficiently accessible and transportable and the weight of the redundant information is reduced.

The main innovative aspects of the disclosed invention are the following.

1 Sequence reads are classified and partitioned into data classes according to the results of the alignment with respect to reference sequences. Such classification and partitioning enables the selective access to encoded data according to criteria related to the alignment results and to the matching accuracy.

2 The classified sequence reads and the associated metadata are represented by genomic descriptors organized in blocks with homogeneous statistical properties enabling the definition of distinct information sources characterized by a low information entropy.

3 The possibility of modeling each separated information source with distinct source model adapted to the statistical characteristics of each class and the possibility of changing the source model within each class of reads and within each descriptor block for each separately accessible data units (Access Units). The adoption of the appropriate transformation, binarization and context adaptive probability models and associated entropy coders according to the statistical properties of each source model.

4 The definition of correspondences and dependencies among the descriptors blocks to enable the selective access to the sequencing data and associated metadata without the need to decode all the descriptors blocks if not all information is required.

5 The coding of each sequence data class and associated metadata blocks with respect to "pre-existing" (also denoted as "external") reference sequences or with respect to "transformed" reference sequences obtained by applying appropriate transformations to "pre-existing" reference sequences so as to reduce the entropy of the descriptors blocks information sources. Said descriptors represent the reads partitioned into the different data classes. Following any encoding of reads using the corresponding descriptors with reference to a "pre-existing" reference or "transformed" "pre-existing" reference sequence, the occurrence of the various mismatches can be used to define the appropriate transformations to the reference sequences in order to find a final coded representation with low entropy and achieve higher compression efficiency.

6 The construction of one or more reference sequences (also referred to as "internal" references to distinguish from the "pre-existing" also referred here as "external" reference sequences) used to encode the class of reads that present a degree of matching accuracy with respect to the pre-existing reference sequences not satisfying a set of constraints. Such constraints are set with the objective that the coding costs of representing in compressed form the class of reads aligned with respect to the "internal" reference sequences and the cost of representing the "internal" reference sequences themselves, is lower than encoding the unaligned class of reads verbatim, or using the "external" reference sequences without or with transformations.

7 The transmission of the configuration parameters governing the process of both encoding and decoding by means of data structures embedded in the compressed genomic data in the form of header information. Such configuration parameters can be updated during the encoding process in order to improve the compression performance. Such updates are conveyed in the compressed content in the form of updated configuration data structures.

In the following, each of the above aspects will be further described in details.

**Classification of the sequence reads according to matching rules**

The sequence reads generated by sequencing machines are classified by the disclosed invention into six different "classes" according to the matching results of the alignment with respect to one or more "pre-existing" reference sequences.

When aligning a DNA sequence of nucleotides with respect to a reference sequence the following cases can be identified:

1. A region in the reference sequence is found to match the sequence read without any error (i.e. perfect mapping). Such sequence of nucleotides is referenced to as "perfectly matching read" or denoted as "Class P".

2. A region in the reference sequence is found to match the sequence read with a type and a number of mismatches determined only by the number of positions in which the sequencing machine generating the read was not able to call any base (or nucleotide). Such type of mismatches are denoted by an "N", the letter used to indicate an undefined nucleotide base. In this disclosure this type of mismatch are referred to as "n type" mismatch. Such sequences belong to "Class N" reads. Once the read is classified to belong to "Class N" it is useful to limit the degree of matching inaccuracy to a given upper bound and set a boundary between what is considered a valid matching and what it is not. Therefore, the reads assigned to Class N are also constrained by setting a threshold (MAXN) that defines the maximum number of undefined bases (i.e. bases called as "N") that a read can contain. Such classification implicitly defines the required minimum matching accuracy (or maximum degree of mismatch) that all reads belonging to Class N share when referred to the corresponding reference sequence, which constitutes an useful criterion for applying selective data searches to the compressed data.

3. A region in the reference sequence is found to match the sequence read with types and number of mismatches determined by the number of positions in which the sequencing machine generating the read was not able to call any nucleotide base, if present (i.e. "n type" mismatches), plus the number of mismatches in which a different base, than the one present in the reference, has been called. Such type of mismatch denoted as "substitution" is also called Single Nucleotide Variation (SNV) or Single Nucleotide Polymorphism (SNP). In this disclosure this type of mismatch is also referred to as "s type" mismatch. The sequence read is then referenced to as "M mismatching reads" and assigned to "Class M". Like in the case of "Class N", also for all reads belonging to "Class M" it is useful to limit the degree of matching inaccuracy to a given upper bound, and set a boundary between what is considered a valid matching and what it is not. Therefore, the reads assigned to Class M are also constrained by defining a set of thresholds, one for the number "n" of mismatches of "n type" (MAXN) if present, and another for the number of substitutions "s" (MAXS). A third constraint is a threshold defined by any function of both numbers "n" and "s", f(n,s). Such third constraint enables to generate classes with an upper bound of matching inaccuracy according to any meaningful selective access criterion. For instance, and not as a limitation, f(n,s) can be (n+s)1/2 or (n+s) or any linear or non-linear expression that sets a boundary to the maximum matching inaccuracy level that is admitted for a read belonging to "Class M". Such boundary constitutes a very useful criterion for applying the desired selective data searches to the compressed data when analyzing sequence reads for various purposes because it makes possible to set a further boundary to any possible combination of the number of "n type" mismatches and "s type" mismatches (substitutions) beyond the simple threshold applied to the one type or to the other.

4. A fourth class is constituted by sequencing reads presenting at least one mismatch of any type among "insertion", "deletion" (a.k.a. *indels*) and "clipped", plus, if present, any mismatches type belonging to class N or M. Such sequences are referred to as "I mismatching reads" and assigned to "Class I". Insertions are constituted by an additional sequence of one or more nucleotides not present in the reference, but present in the read sequence. In this disclosure this type of mismatch is referred to as "i type" mismatch. In literature when the inserted sequence is at the edges of the sequence it is also referred to as "soft clipped" (i.e. the nucleotides are not matching the reference but are kept in the aligned reads contrarily to "hard clipped" nucleotides which are discarded). In this disclosure this type of mismatch is referred to as "c type" mismatch. Keeping or discarding nucleotides is a decisions taken by the aligner stage and not by the classifier of reads disclosed in this invention which receives and processes the reads as they are determined by the sequencing machine or by the following alignment stage. Deletion are "holes" (missing nucleotides) in the read with respect to the reference. In this disclosure this type of mismatch is referred to as "d type" mismatch. Like in the case of classes "N" and "M" it is possible and appropriate to define a limit to the matching inaccuracy. The definition of the set of constraints for "Class I" is based on the same principles used for "Class M" and is reported in Table 1 in the last table lines. Beside a threshold for each type of mismatch admissible for Class I data, a further constraint is defined by a threshold determined by any function of the number of the mismatches "n", "s", "d", "i" and "c", $w(n,s,d,i,c)$. Such additional constraint make possible to generate classes with an upper bound of matching inaccuracy according to any meaningful user defined selective access criterion. For instance, and not as a limitation, $w(n,s,d,i,c)$ can be $(n+s+d+i+c)1/5$ or $(n+s+d+i+c)$ or any linear or non-linear expression that sets a boundary to the maximum matching inaccuracy level that is admitted for a read belonging to "Class I". Such boundary constitutes a very useful criterion for applying the desired selective data searches to the compressed data when analyzing sequence reads for various purposes because it enables to set a further boundary to any possible combination of the number of mismatches admissible in "Class I" reads beyond the simple threshold applied to each type of admissible mismatch.

5. A fifth class includes all reads that do not find any mapping considered valid (i.e not satisfying the set of matching rules defining an upper bound to the maximum matching inaccuracy as specified in Table 1) for each data class when referring to the reference sequence. Such sequences are said to be "Unmapped" when referring to the reference sequences and are classified as belonging to the "Class U".

6. In case of paired-end reads, a sixth class is defined in which one read of the pair cannot map at any position of the reference genome (i.e. belongs to Class U) and the other read belongs to one of the P, N, M, I classes. Such class is named "Class HM" from Half-Mapped.

## Classification of read pairs according to matching rules

The classification specified in the previous section concerns single sequence reads. In the case of sequencing technologies that generates read in pairs (i.e. Illumina Inc.) in which two reads are known to be separated by an unknown sequence of variable length, it is appropriate to consider the classification of the entire pair to a single data class. A read that is coupled with another is said to be its "mate".

If both paired reads belong to the same class the assignment to a class of the entire pair is the following: the entire pair is assigned to the same class for any class (i.e. P, N, M, I, U). In the case the two reads belong to a different class, but none of them belongs to the "Class U", then the entire pair is assigned to the class with the highest priority defined according to the following expression:

$$P < N < M < I$$

in which "Class P" has the lowest priority and "Class I" has the highest priority.

In case only one of the reads belongs to "Class U" and its mate to any of the Classes P, N, M, I a sixth class is defined as "Class HM" which stands for "Half Mapped".

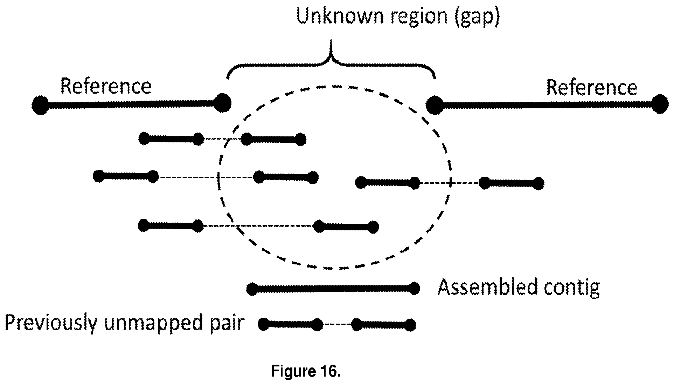

The definition of such specific class of reads and the associated assignment rules are motivated by the fact that such data is used for attempting to determine gaps or unknown regions of reference genomes (a.k.a. little known or unknown regions). Such regions are reconstructed by mapping pairs at the edges using the pair read that can be mapped on the known regions. The unmapped mate is then used to build the so called "contigs" of the unknown region as it is shown in figure 16. Therefore providing a selective access to only such type of read pairs greatly reduces the associated computation burden enabling much efficient processing of such data originated by large amounts of data sets that using the state of the art solutions would require to be entirely inspected.

The table below summarizes the matching rules applied to reads in order to define the class of data each read belongs to. The rules are defined in the first five columns of the table in terms of presence or absence of type of mismatches (n, s, d, i and c type mismatches). The sixth column provide rules in terms of maximum threshold for each mismatch type and any function $f(n,s)$ and $w(n,s,d,i,c)$ of the possible mismatch types.

**Table 1. Type of mismatches and set of constrains that each sequence reads must satisfy to be classified in the data classes defined in this invention disclosure.**

| Number and types of mismatches found when matching a read with a reference sequence | | | | | Set of matching accuracy constraints | Class |
|---|---|---|---|---|---|---|
| Number of unknown bases ("N") | Number of substitutions | Number of deletions | Number of Insertion s | Number of clipped bases | | |
| 0 | 0 | 0 | 0 | 0 | 0 | P |
| $n > 0$ | 0 | 0 | 0 | 0 | $n \leq$ MAXN | N |
| | | | | | $n >$ MAXN | U |
| $n \geq 0$ | $s > 0$ | 0 | 0 | 0 | $n \leq$ MAXN and $s \leq$ MAXS and $f(n,s) \leq$ MAXM | M |
| | | | | | $n >$ MAXN or $s >$ MAXS or $f(n,s) >$ MAXM | U |
| $n \geq 0$ | $s \geq 0$ | $d \geq 0$ * | $i \geq 0$* | $c \geq 0$* | $n \leq$ MAXN and $s \leq$ MAXS and $d \leq$ MAXD and $i \leq$ MAXI and $c \leq$ MAXC $w(n,s,d,i,c) \leq$ MAXTOT | I |
| | | **\*At least one mismatch of type d, i, c must be present (i.e. d>0 or i>0 or c>0)** | | | | |
| | | $d \geq 0$ | $i \geq 0$ | $c \geq 0$ | $n >$ MAXN or $s >$ MAXS or $d >$ MAXD or $i >$ MAXI or $c >$ MAXC $w(n,s,d,i,c) >$ MAXTOT | U |

***Matching rules partition of sequence read data Classes N, M and I into subclasses with different degrees of matching accuracy***

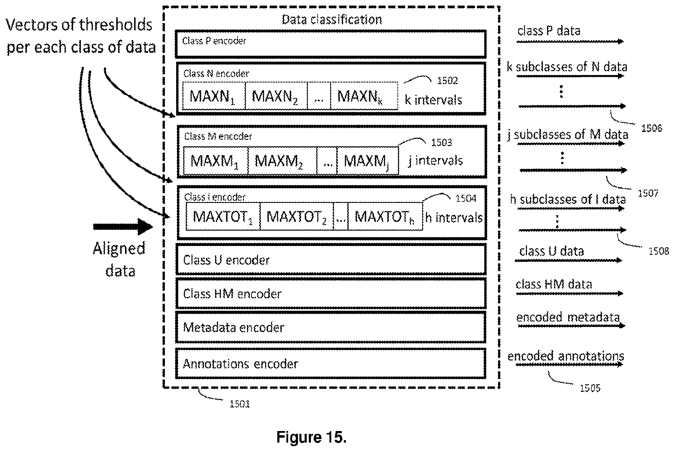

The data classes of type N, M and I as defined in the previous sections can be further decomposed into an arbitrary number of distinct sub-classes with different degrees of matching accuracy. Such option is an important technical advantage in providing a finer granularity and as consequence a much more efficient selective access to each data class. As an example and not as a limitation, to partition the Class N into a number k of subclasses (Sub-Class $N_1$, ..., Sub-Class $N_k$) it is necessary to define a vector with the corresponding components $MAXN_1$, $MAXN_2$, ..., $MAXN_{(k-1)}$, $MAXN_{(k)}$, with the condition that $MAXN_1 < MAXN_2 < ... < MAXN_{(k-1)} < MAXN$ and assign each read to the lowest ranked sub-class that satisfy the constrains specified in Table 1 when evaluated for each element of the vector. This is shown in figure 15 where a data classification unit 1501 contains Class P, N, M, I, U, HM encoder and encoders for annotations and metadata. Class N encoder is configured with a vector of thresholds, $MAXN_1$ to $MAXN_k$ 1502 which generates k subclasses of N data (1506).

In the case of the classes of type M and I the same principle is applied by defining a vector with the same properties for MAXM and MAXTOT respectively and use each vector components as threshold for checking if the functions f(n,s) and w(n,s,d,l,c) satisfy the constraint. Like in the case of sub-classes of type N, the assignment is given to the lowest sub-class for which the constraint is satisfied. The number of sub-classes for each class type is independent and any combination of subdivisions is admissible. This is shown in figure 15 where a Class M encoder 1503 and a Class I encoder 1504 are configured respectively with a vector of thresholds $MAXM_1$ to $MAXM_j$ and $MAXTOT_1$ to $MAXTOT_h$ . The two encoders generate respectively j subclasses of M data (1507) and h subclasses of I data (1508).

When two reads in a pair are classified in the same sub-class, then the pair belongs to the same sub-class.

When two reads in a pair are classified into sub-classes of different classes, then the pair belongs to the sub-class of the class of higher priority according to the following expression:

$$N < M < I$$

where N has the lowest priority and I has the highest priority.

When two reads belong to different sub-classes of one of classes N or M or I, then the pair belongs to the sub-class with the highest priority according to the following expressions:

$$N_1 < N_2 < \ldots < N_k$$

$$M_1 < M_2 < \ldots < M_j$$

$$I_1 < I_2 < \ldots < I_h$$

where the highest index has the highest priority.

*Transformations of the "external" reference sequences*

The mismatches found for the reads classified in the classes N, M and I can be used to create "transformed" references to be used to compress more efficiently the read representation.

Reads classified as belonging to the Classes N, M or I (with respect to the "pre-existing" (i.e."external") reference sequence denoted as $RS_0$) can be coded with respect to the "transformed" reference sequence $RS_1$ according to the occurrence of the actual mismatches with the "transformed" reference. For example if $read^M_{in}$ belonging to Class M (denoted as the $i^{th}$ read of class M) containing mismatches with respect to the reference sequence $RS_n$, then after "transformation" $read^M_{in} = read^P_{i(n+1)}$ can be obtained with $A(Ref_n) = Ref_{n+1}$ where A is the transformation from reference sequence $RS_n$ to reference sequence $RS_{n+1}$.

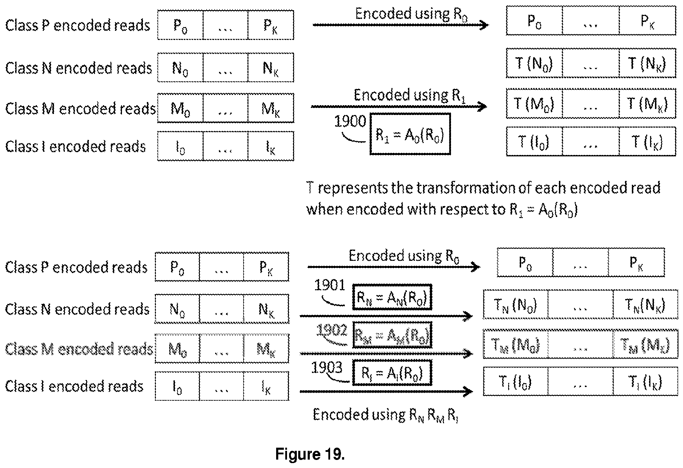

Figure 19 shows an example on how reads containing mismatches (belonging to Class M) with respect to reference sequence 1 ($RS_1$) can be transformed into perfectly matching reads with respect to the reference sequence 2 ($RS_2$) obtained from $RS_1$ by modifying the bases corresponding to the mismatch positions. They remain classified and they are coded together the other reads in the same data class access unit, but the coding is done using only the descriptors and descriptor values needed for a Class P read. This transformation can be denoted as: $RS_2 = A(RS_1)$

When the representation of the transformation A which generates $RS_2$ when applied to $RS_1$ plus the representation of the reads versus $RS_2$ corresponds to a lower entropy than the representation of the reads of class M versus $RS_1$, it is advantageous to transmit the representation of the transformation A and the corresponding representation of the read versus $RS_2$ because an higher compression of the data representation is achieved.

The coding of the transformation A for transmission in the compressed bitstream requires the definition of two additional syntax elements as defined in the table below.

| Syntax elements | Semantic | Comments |
|---|---|---|
| rftp | Reference transformation position | position of difference between reference and contig used for prediction |
| rftt | Reference transformation type | type of difference between reference and contig used for prediction. Same syntax described for the mmtype descriptor defined below in this disclosure. |

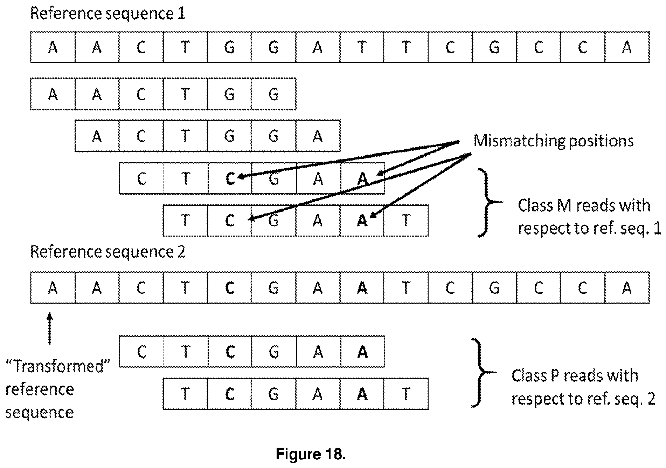

Figure 18 shows an example on how a reference transformation is applied to reduce the number of mismatches to be coded on the mapped reads.

It has to be observed that, in some cases the transformation applied to the reference:

- May introduce mismatches in the representations of the reads that were not present when referring to the reference before applying the transformation.

- May modify the types of mismatches, a read may contain A instead of G while all other reads contain C instead of G), but mismatches remain in the same position.
- Different data classes and subsets of data of each data class may refer to the same "transformed" reference sequences or to reference sequences obtained by applying different transformations to the same pre-existing reference sequence.

Figure 19 further shows an example of how reads can change the type of coding from a data class to another by means of the appropriate set of descriptors (e.g. using the descriptors of a Class P to code a read from Class M) after a reference transformation is applied and the read is represented using the "transformed" reference. This occurs for example when the transformation changes all bases corresponding to the mismatches of a read in the bases actually present in the read, thus virtually transforming a read belonging to Class M (when referring to the original non "transformed" reference sequence) into a virtual read of Class P (when referring to the "transformed" reference). The definition of the set of descriptors used for each class of data is provided in the following sections.

Figure 19 shows how the different classes of data can use the same "transformed" reference $R_1 = A_0(R_0)$ (1900) to re-encode the reads, or different transformations $A_N$ (1901), $A_M$ (1902), A, (1903) can be separately applied to each class of data to produce different reference genomes $R_N$, $R_M$, $R_I$

### Genomic Dataset Header

Once the classification of reads is completed with the definition of the Classes, further processing consists in defining a set of distinct syntax elements which represent the remaining information enabling the reconstruction of the read sequence when represented as being mapped on a given reference sequence. The data structure of these syntax elements requires the storage of global parameters and metadata to be used by the decoding engine. These data are structured in a **Genomic Dataset Header** described in the table below. A dataset is defined as the ensemble of coding elements needed to reconstruct the genomic information related to a single genomic sequencing run and all the following analysis. If the same genomic sample is sequenced twice in two distinct runs, the obtained data will be encoded in two distinct datasets.

**Table 2. Genomic Dataset Header.**

| Element | Description |
|---|---|
| dataset_header { | |
| dataset_group_ID | identifier of Dataset Group containing the Dataset including this dataset_header. |
| dataset_ID | identifier of the Dataset. |
| major_brand | the brand identifier, identifying the data (compression) format specification the Dataset complies with |
| minor_version | minor version number of the data (compression) format specification the Dataset complies with |
| unmapped_indexing_flag | flag indicating the presence of indexed Access Units of Class U |
| byte_offset_size_flag | unmber of bits used to index the Access Units |
| non_overlapping_AU_range | if set, all Access Units in the Dataset have non-overlapping ranges. |
| block_header_flag | if set, all Blocks composing the Dataset are preceded by a Block Header |
| if (block_header_flag) { | |
| mit_flag | if set, the indexing tool called Master Index Table is present |
| cc_mode_flag | if set, two Access Units of a class cannot be separated by Access Units of a different class in the storage device. |
| block_start_code_prefix_ flag | if set, all Access Unit Headers start by the three bytes 0x000001 |
| } | |
| else { | |

(continued)

| Element | Description |
|---|---|
| ordered_blocks_flag | if set, Access Unit are ordered by increasing value of the mapping position of the first read encoded in each Access Unit |
| } | |
| seq_count | number of reference sequences used in this Dataset |
| reference ID | unique identification number of the reference genome used in the Dataset |
| for (seq=0;seq<seq_count; seq++) { | |
| seq_ID[seq] | reference sequence identifier |
| } | |
| for (seq=0;seq<seq_count; seq++) { | |
| seq_blocks[seq] | number of Access Units per reference sequence |
| } | |
| dataset_type | 0 = non-aligned content; 1 = aligned content; 2 = reference |
| num_classes | number of data classes encoded in the Dataset |
| for (ci=0;ci<num_classes; ci++) { | |
| clid[ci] | identifies the class of data carried by the Access Unit |
| num_descriptors[ci] | maximum number of Descriptors per Class encoded in the Dataset |
| for (desc_ID=0;desc_ID<num_descriptors[ci];desc_ID++) { | |
| descriptor ID[ci][desc_ID] | unambiguous genomic descriptor identifier as defined in Table 1 |
| } | |
| alphabet_ID | identifier of the alphabet used to encode the Class U data signatures |
| num_U_clusters | number of clusters of class U reads |
| U_signature_constant_length | 1 = constant length; 0 = variable length |
| U_signature_size | size in bits of each integer representing an encoded signature |
| if (U_signature_constant_length){ | |
| U_signature_length | length of class U clusters signatures as number of nucleotides |
| } | |
| num_U_access_units | total number of Access Units in the Dataset containing encoded data of class U |
| multiple_alignment_flag | if set it indicates the presence of multiple alignments in the Dataset |
| multiple_signature_base | default number of signatures for reads of class U |
| tflag[0] | flag indicating the presence of a threshold thres for the corresponding reference sequence |
| thres[0] | |
| for (i=1;i<seq_count;i++) { | |
| tflag[i] | flag indicating the presence of a threshold thres for the corresponding reference sequence |

(continued)

| Element | Description |
|---|---|
| if(tflag[i] == 1) | |
| thres[i] | threshold indicating the maximum difference between the access unit covered region and the access unit range |
| else /* tflag[i] == 0 */ | |
| thres[i] = thres[i-1] | if tflag is not set for a reference sequence, the previous value apply |
| } | |
| } | |

## Genomic Descriptors

A sequence read (i.e. a DNA segment) referred to a given reference sequence can be fully expressed optionally using a subset formed of various combinations of the following descriptors:

**Table 3. Genomic descriptors and their meaning**

| descriptor_id | short name | description |
|---|---|---|
| 1 | pos | the mapping position of a read on a reference sequence |
| 2 | pair | in case of paired end reads, it represents how the reads are paired |
| 3 | rlen | the length of a sequence read |
| 4 | rcomp | the DNA or RNA strand the reads was mapped on |
| 5 | mmpos | the position of mismatches (i.e. substitutions, deletion and insertions ) in aligned reads with respect to reference sequences |
| 6 | mmtype | the types of mismatches with respect to reference sequences at the associated positions |
| 7 | clips | the bases that could not be mapped on the reference sequence by the mapping procedure and either kept ("soft clipped" bases) or discarded ("hard clipped" bases) |
| 8 | flags | mapping flags to enable the aligner to further specify the result of the mapping process such as if the sequence read is a PCR or optical duplicate |
| 9 | mmap | the multiple mapping positions that are associated to a single read or read pair by the mapping procedure |
| 10 | msar | the identification of the existence of spliced reads (i.e. reads that when split in chunks find mapping positions with higher degrees of matching accuracy than when they are mapped as single contiguous read mapped on a single position on a reference sequence) |
| 11 | ureads | the representation of sequence reads that cannot be mapped at any position of the reference sequence with specified degrees of matching accuracy, |
| 12 | rtype | descriptor used to signal the subset of descriptors used to encode sequence reads that cannot be mapped at any position of the reference sequence with specified degrees of matching accuracy |
| 13 | rftp | the position of mismatches between a contig and a reference sequence. Positions of mismatches are terminated by a special terminator character. |
| 14 | rftt | the type of mismatches between a contig and a reference sequence. |
| 15 | rgroup | label associated to each compressed sequence read used to create group of reads sharing the same label |

(continued)

| descriptor_id | short name | description |
|---|---|---|
| 16 | mscore | score per alignment. It is used to represent mapping/alignment score per read generated by genomic sequence reads aligners. |

For class U, the descriptor **clips** identifies those parts of the reads (typically the edges) that do not match, with a specified set of matching accuracy constraints, with the "internal" references. Descriptor **ureads** is used to encode verbatim the reads that cannot be mapped on any available reference being it a pre-existing ( i.e. "external" like an actual reference genome) or an "internal" reference sequence.

This classification creates groups of descriptors (syntax elements) that can be used to univocally represent genome sequence reads. The table below summarizes the syntax elements needed for each class of reads aligned with "external" (i.e. "pre-existing") or "internal" (i.e. "constructed") references. The asterisk "*" indicates mandatory descriptors always present in all encoded read for each class.

**Table 4.Genomic descriptors necessary to represent each class of data.**

| descriptors | | data classes | | | | | |
|---|---|---|---|---|---|---|---|
| descriptor_id | short name | P | N | M | I | U | HM |
| 1 | pos | X* | X* | X* | X* | X* | X* |
| 2 | pair | X | X | X | X | | |
| 3 | rlen | X | X | X | X | X | X |
| 4 | rcomp | X* | X* | X* | X* | X* | X* |
| 5 | mmpos | | X* | X* | X* | X* | X |
| 6 | mmtype | | | | X* | X* | X |
| 7 | clips | | | | X* | X* | X |
| 8 | flags | X* | X* | X* | X* | X* | X* |
| 9 | mmap | | | | X | | X |
| 10 | msar | | | | X | | X |
| 11 | ureads | | | | X | X | X* |
| 12 | rtype | | | | | X | |
| 13 | rftp | X | X | X | X | X | X |
| 14 | rftt | X | X | X | X | X | X |
| 15 | rgroup | X | X | X | X | X | X |
| 16 | mscore | X | X | X | X | | X |

Reads belonging to class P are characterized and can be perfectly reconstructed by only a position, a reverse complement information and an offset between mates in case they have been obtained by a sequencing technology yielding mated pairs, some flags and a read length. The next section further details how these descriptors are defined for classes P, N, M and I while for class U they are described in a following section

Class HM is applied to read pairs only and it is a special case for which one read belongs to class P, N, M or I and the other to class U.

### pos descriptor

The **pos** descriptor is used to calculate the absolute mapping position on a Reference Sequence of the left-most mapped base of a Genomic Record. The value of each **pos** descriptor represents the difference between the coordinates, on the Reference Sequence, of the left-most mapping base of a Genomic Record and the previous one.

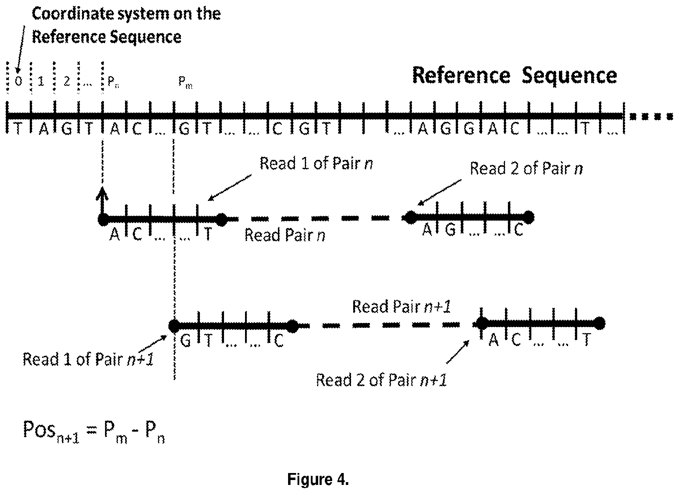

Figure 4 shows an example of calculation of the **pos** descriptor for a mapped read pair.

The first value of the **pos** descriptor in each coded Block is always 0 since no differential coding is possible for the first mapped read or read pair coded in an Access Unit. The absolute position of the first mapped read or read pair coded in an Access Unit is contained in the Access Unit Header.

The absolute position on the Reference Sequence of the left-most mapped base of the $n^{th}$ Genomic Record is therefore calculated as:

$$p_n = p_0 + \sum_{i=1}^{n} pos_i$$

where $p_0$ is the mapping value retrieved from the Access Unit Header for the first Genomic Record in the Access Unit.

In order to calculate the absolute position on a Reference Sequence of a base the following formula applies:

$$p = p_{start} + n_{del} - n_{ins} + d_{start} + delta$$

where

- $p$       is the absolute position on the Reference Sequence of the base
- $p_{start}$    is the mapping position of the Genomic Record covering the base
- $n_{ins}$    is the number of inserted bases preceding the base in the same Genomic Record
- $n_{del}$    is the number of deleted bases preceding the base in the same Genomic Record
- $d_{start}$    is the offset of the base in the Genomic Record from the Genomic Record Position
- delta    is the (signed) pairing distance between two reads in a read pair. This has to be used only for positions in the second read of the Genomic Record.

NOTE In case of paired end reads, when calculating the offset $d_{start}$ from the Genomic Record Position, the two reads are considered contiguous. The relative reads position is taken into account when adding **delta.**

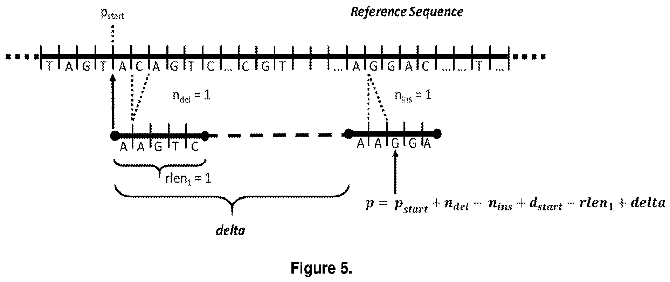

An example of calculation of the mapping position p for one base on a Reference Sequence is shown in figure 5.

### rcomp descriptor

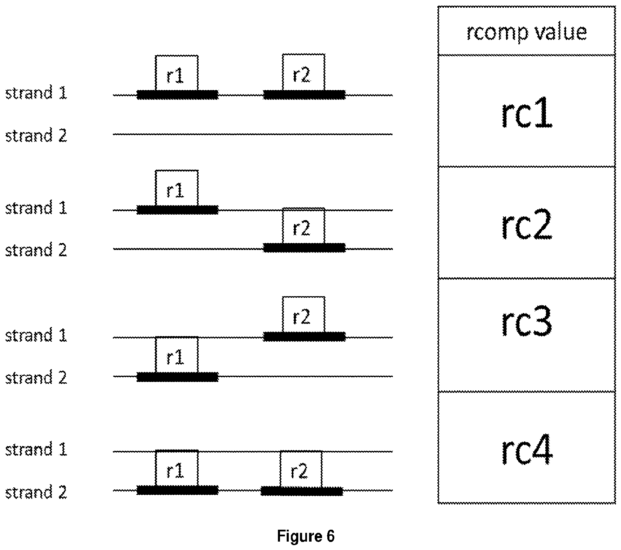

The **rcomp** descriptor conveys information about the *strandedness* of reads. Each bit of a decoded **rcomp** descriptor is a flag indicating if the read is on the forward (bit set to 0) or reverse (bit set to 1) strand. Figure 6 shows values and semantics of the **rcomp** descriptor for paired end reads. In the figure r1 is read 1 and r2 is read 2. According to the mapping position of each read, the rcomp descriptor can have four different values.

**Table 5. Values and meaning of the rcomp descriptor.**

| Value | | Semantics | |
|---|---|---|---|
| | | Single read | Paired reads |
| rc1 | 0x00 | read on forward strand | both reads on forward strand (strand 1) |
| rc2 | 0x01 | read on reverse strand | first read on forward strand, second on reverse strand |
| rc3 | 0x02 | reserved | first read on reverse strand, second on forward strand |
| rc4 | 0x03 | reserved | both reads on reverse strand (strand 2) |
| | 0x04 .. 0xff | reserved | reserved |

### flags descriptor

The flag descriptor is a set of flags as described in Table 6.

**Table 6. Semantics for each bit of the flag descriptor.**

| bit position from LSB | Semantics |
|---|---|
| 0 | read is PCR or optical duplicate |
| 1 | read fails platform/vendor quality checks |
| 2 | read mapped in proper pair |
| 3 | reserved |
| 4 | reserved |
| 5 | reserved |
| 6 | reserved |
| 7 | reserved |

***mmpos***

The **mmpos** descriptor represents the position, within a read or read pairs, of a mismatch with respect to the reference sequence. The position is represented as a distance from the position of the previous mismatch in the Genomic Record. The position of the first mismatch is represented as the distance from the left-most mapped base in the Genomic Record.

In case of paired reads, or in general records containing more than one genomic segment, the gaps between consecutive segments are not considered in the calculation of distances between consecutive mismatches.

If the encoded pair contains both read 1 and read 2, the positions of mismatches in read 2 are offset by the length of read 1. For example in case of reads with constant length equal to 100, if the first mismatches in the pair is in read 2 at position 44, the first **mmpos** descriptor decoded for this Genomic Record assumes the value 144.

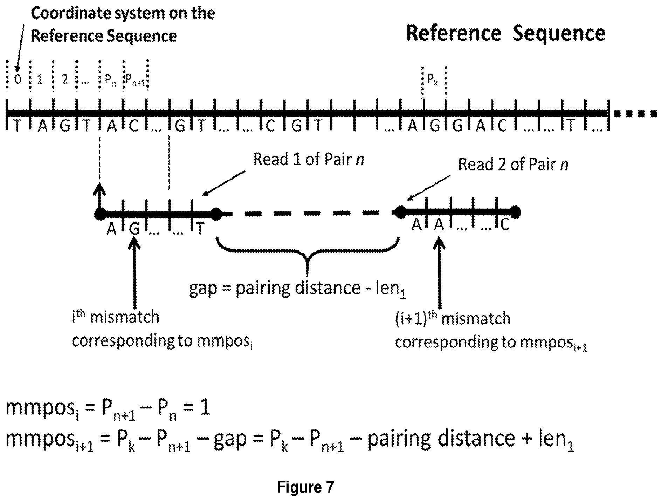

If the described pair is missing read 1 (either because it is encoded in another block, or because read 2 is unpaired), the mismatch positions are not offset by the length of read 1. For example in case of fixed read length 100, if the first mutation in read 2 is at position 44, but read 2 is unpaired, the first **mmpos** descriptor decoded for this Genomic Record assumes the value 44. Each **mmpos** descriptor is associated to an **mmtype** descriptor representing the type of mismatch occurring in the decoded read or read pair at the position calculated using **mmpos.** An example of how to calculate mismatches positions in a read pair is provided in figure 7 where $len_1$ is the length of read1.

The absolute position on a Reference Sequence of the $i^{th}$ mismatch in a Genomic Record shall be calculated as shown in Table 7.

**Table 7. How to calculate the absolute position of mismatches in a Genomic Record.**

| | **First mismatch** | $i^{th}$ **mismatch (i > 0)** |
|---|---|---|
| **Single read** | $mmabs_0 = p_0 + mmpos_0$ | $mmabs_i = mmabs_{i-1} + mmpos_i$ |
| | | |
| **Read pair** | | |
| first mismatch in read 1 | $mmabs_0 = p_0 + mmpos_0$ | if(( $\Sigma mmpos_i < len_1 \parallel$ $(\Sigma mmpos_i \geq len_1$ && $\Sigma mmpos_{i-1} < len_1))$ $mmabs_i = mmabs_{i-1} + mmpos_i$ else $mmabs_i = mmabs_{i-1} + mmpos_i + delta$ |
| first mismatch in read 2 | $mmabs_0 = p_0 + mmpos_0 + delta$ | |

In Table 7 the following variables are defined:

- $mmabs_i$ is the absolute position in the Reference Sequence of the $i^{th}$ mismatch in the read or read pair
- $mmpos_i$ is the $i^{th}$ value of the **mmpos** descriptor in the Genomic Record
- $len_1$ is the length of read 1 in a read pair
- delta is the pairing distance between read 1 and read 2 calculated as defined for the **pair** descriptor and shown in figure 5

Sequences of **mmpos** descriptors referring to a Genomic Record are terminated with a reserved terminator value which cannot be interpreted as mismatch position.

*mmtype*

The **mmtype** descriptor specifies the type of mismatch occurring in the decoded read at the position calculated using the associated **mmpos** descriptor.

The mmtype descriptor does not have a reserved value for the terminator since each Genomic Record shall contain the same number of **mmtype** and **mmpos** descriptors.

Table 8 lists the values and corresponding semantics of the **mmtype** descriptor according to the used alphabet.

**Table 8. mmtype descriptor values and semantics according to the used alphabet *clips***

| Value | Semantics | | | |
|---|---|---|---|---|
| | alphabet_id = 0 | alphabet_id = 1 | alphabet_id = 2 | alphabet_id = 3 |
| 0x00 | Substitute with 'A' | Substitute with 'A' | Substitute with 'A' | Substitute with 'A' |
| 0x01 | Substitute with 'C' | Substitute with 'C' | Substitute with 'C' | Substitute with 'C' |
| 0x02 | Substitute with 'G' | Substitute with 'G' | Substitute with 'G' | Substitute with 'G' |
| 0x03 | Substitute with 'T' | Substitute with 'T' | Substitute with 'U' | Substitute with 'U' |
| 0x04 | Substitute with 'N' | Substitute with 'N' | Substitute with 'N' | Substitute with 'N' |
| 0x05 | Deletion | Deletion | Deletion | Deletion |
| 0x06 | Insert 'A' | Substitute with 'R' | Insert 'A' | Substitute with 'R' |
| 0x07 | Insert 'C' | Substitute with 'Y' | Insert 'C' | Substitute with 'Y' |
| 0x08 | Insert 'G' | Substitute with Is, | Insert 'G' | Substitute with 'S' |
| 0x09 | Insert 'T' | Substitute with 'W' | Insert 'U' | Substitute with 'W' |
| 0x0A | Insert 'N' | Substitute with 'K' | Insert 'N' | Substitute with 'K' |
| 0x0 B | N/A | Substitute with 'M' | N/A | Substitute with 'M' |
| 0x0C | N/A | Substitute with 'B' | N/A | Substitute with 'B' |
| 0x0D | N/A | Substitute with 'D' | N/A | Substitute with 'D' |
| 0x0E | N/A | Substitute with 'H' | N/A | Substitute with 'H' |
| 0x0F | N/A | Substitute with 'V' | N/A | Substitute with 'V' |
| 0x10 | N/A | Substitute with '-' | N/A | Substitute with '-' |
| 0x11 | N/A | Insert 'A' | N/A | Insert 'A' |
| 0x12 | N/A | Insert 'C' | N/A | Insert 'C' |
| 0x13 | N/A | Insert 'G' | N/A | Insert 'G' |
| 0x14 | N/A | Insert 'T' | N/A | Insert 'T' |
| 0x15 | N/A | Insert 'N' | N/A | Insert 'N' |
| 0x16 | N/A | Insert 'R' | N/A | Insert 'R' |
| 0x17 | N/A | Insert 'Y' | N/A | Insert 'Y' |
| 0x18 | N/A | Insert 'S' | N/A | Insert 'S' |
| 0x19 | N/A | Insert 'W' | N/A | Insert 'W' |
| 0x1A | N/A | Insert 'K' | N/A | Insert 'K' |
| 0x1B | N/A | Insert 'M' | N/A | Insert 'M' |
| 0x1C | N/A | Insert 'B' | N/A | Insert 'B' |

(continued)

| Value | Semantics | | | |
|---|---|---|---|---|
| | alphabet_id = 0 | alphabet_id = 1 | alphabet_id = 2 | alphabet_id = 3 |
| 0x1D | N/A | Insert 'D' | N/A | Insert 'D' |
| 0x1E | N/A | Insert 'H' | N/A | Insert 'H' |
| 0x1F | N/A | Insert 'V' | N/A | Insert 'V' |
| 0x20 | N/A | Insert '-' | N/A | Insert '-' |

The **clips** descriptor is used to represent clipped bases (a.k.a. soft or hard clips) in mapped reads or read pairs. This descriptor encodes soft clips as sequences of ASCII characters with additional elements to identify the position of the clipped bases in the read or read pair. In case of hard clips only the position and the number of clipped bases is encoded. Each descriptor contains a Genomic Record identifier followed by information related to the clipped bases position in the Genomic Record and the actual clipped bases in case of soft clips.

Syntax and semantics of the **clips** descriptor is provided in Table 9 and Table 10.

**Table 9. Fields composing the clips descriptor.**

| Size in bytes | Semantics | Remarks |
|---|---|---|
| Soft clips | | |
| 4 | Genomic Record identifier (sequential counter of the Genomic Records encoded in the current Access Unit) | repeated up to 2N times per Genomic Record where N is the number of segments in the Genomic Record. |
| 1 | flag indicating the position (0x00 start of read1, 0x01 = end of read 1, 0x02 = start of read 2, 0x03 = end of read2) of clipped bases | |
| M | sequence of M bases encoded as ASCII characters. | |
| 1 (optional) | soft clips terminator (0xfe) present only if in the same Genomic Record more than 1 soft clips sequence is present | If K is the number of clipped segments, this is present only after the first K-1 sections |
| 1 | Genomic Record terminator (0xff) | present only after the K$^{th}$ (last) sections |
| Hard clips | | |
| 4 | Genomic Record identifier (sequential counter of the Genomic Records encoded in the current Access Unit) | repeated up to 2N times per Genomic Record where N is the number of |
| 1 | flag indicating the position (0x04 start of read1, 0x05 = end of read 1, 0x06 = start of read 2, 0x07 = end of read2) of clipped bases | segments in the Genomic Record. |
| 4 | number of hard clipped bases | |
| 1 (optional) | terminator (0xfe) present only if in the same Genomic Record more than 1 clipped sequence is present | If K is the number of clipped segments, this is present only after the first K-1 sections |
| 1 | Genomic Record terminator (0xff) | present only after the K$^{th}$ (last) sections |

**Table 10. Syntax of the clips descriptor.**

| clips() { |
|---|
|     record_id |

(continued)

| |
|---|
| do{ |
|     clips_pos |
|     if(clips_pos <= 3){ |
|         do{ |
|             soft_clipped_base |
|         } while(sclipped_base != Oxff OR sclipped_base != 0xfe) |
|     } |
|     else{ |
|         hard_clipped_bases |
|     } |
| } while(sclipped_base != Oxff) |
| } |

**record_id** is a counter of Genomic Records encoded in the current Access Unit.

**clips_pos** represents the position of the next clipped bases in the read or read pair. Values for positions have the following meaning:

| clips_pos value | semantics |
|---|---|
| 0x00 | soft clips before start of read 1 |
| 0x01 | soft clips after end of read 1 |
| 0x02 | soft clips before start of read 2 |
| 0x03 | soft clips after end of read2 |
| 0x04 | hard clips before start of read 1 |
| 0x05 | hard clips after end of read 1 |
| 0x06 | hard clips before start of read 2 |
| 0x07 | hard clips after end of read2 |

**soft_clipped_base** is one of the symbols of the alphabet identified by **alphabet_id.**

**hard_clipped_bases** represent the number of hard clipped bases at the position indicated by the corresponding **clips_pos;**

*ureads*

The **ureads** descriptor represents verbatim sequence reads as a sequence of ASCII characters belonging to the currently used alphabet identified by **alphabet_id.**

*rlen*

The **rlen** descriptor is used only in case of variable length reads when **reads_length** = 0 in the Parameter Set defined in this disclosure.

A decoded **rlen** descriptor represents the length of the current sequence read as number of bases including soft clips.

*pair*

The information required to reconstruct paired reads is encoded using the **pair** descriptor. The pairing information associating one genomic segment to another can be expressed in three ways:

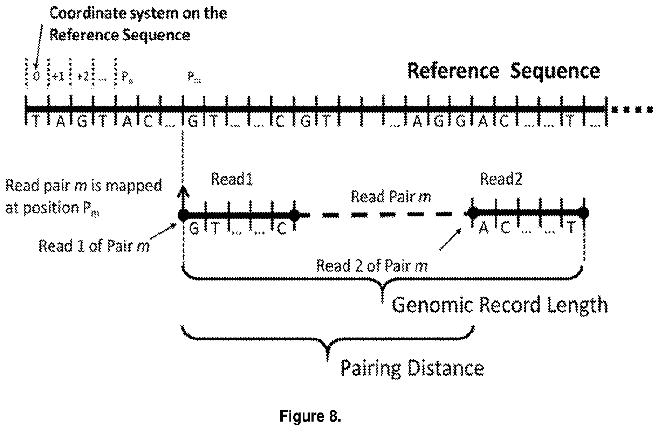

1. if both reads are mapped on the same reference and coded in the same genomic record, the pairing distance is defined as the distance between the left-most mapped base of Read1 and the left-most mapped base of Read2. An example of pairing distance is shown in figure 8.
2. as an absolute mapping position of the second read on the same reference sequence as the first read.
3. as an absolute mapping position of the second read on a different reference sequence than the first read

The pairing information is encoded as described in points 2 and 3 above when the first two bytes of a decoded pair descriptor have one of the values listed in Table 11.

Figure 8 shows how the Genomic Record Length is defined as the number of genomic positions on a reference sequence separating the left-most mapped base from the right-most mapped base of a read or read pair. In case of read pairs this is the number of genomic position on the reference sequence separating the left-most base of read 1 from the right-most base of its mate read 2 when both reads are mapped on the same reference sequence. The "Pairing Distance" is defined as the difference between the left-most position of Read1 and the left-most position of Read2. The "Pairing Distance" is represented as a signed integer valued of the pair descriptor.

When aligning reads to a reference sequence, read 2 can be mapped to a position that is smaller (e.g. to the left) than the mapping position of read 1; in this case, the pairing distance used in case 1 above will be negative. This implies that the information about reads *strandedness* is encoded in the sign of the pairing distance descriptor.

**Table 11. Reserved values for the read distance descriptors signaling how the read pair was encoded**

| Value | Semantics | Followed by |
|---|---|---|
| 0x7ffd | read2 in pair is on the same reference but coded separately | 4 bytes encoding read2 distance as absolute value on the reference |
| 0x8003 | read1 in pair is on the same reference but coded separately | 4 bytes encoding read1 distance as absolute value on the reference |
| 0x7ffe | read2 is on a different reference | 2 bytes with a reference identifier, 4 bytes with absolute position of read2 |
| 0x8002 | read1 is on a different reference | 2 bytes with a reference identifier, 4 bytes with absolute position of read1 |
| 0x7fff | read1 unpaired | N/A |
| 0x8001 | read2 unpaired | N/A |
| 0x8000 | read unpaired | N/A |
| 0x8004 | additional alignment present on another Reference Sequence | 2 bytes with a reference identifier, 4 bytes with absolute position of read1 |

***Reads Distance***

The reads distance is encoded as a 2-bytes signed integer, where:

- the LSB is used to represent the sign (if the sign bit is 0, the number is non-negative, if the sign bit is 1 then the number is negative), and
- the remaining 15 bits are used to represent the absolute value of the pairing distance This approach enables representing pairing distances in a range from -32766 to 32766. In case the reads are separated by a larger gap, the absolute position shall be encoded in the pair descriptor after the special value 0x7ffd or 0x8003 as defined in Table 11 and the two reads are encoded in two separate Genomic Records (i.e. the pair is "split").

***Decoding process for Reads Distance***

The decoding process of the reads distance is shown below:

```
sign = ReadsDistance & 0x0001;
ReadsDistance = ReadsDistance >> 1;
```

```
if (sign) ReadsDistance = -ReadsDistance;
```

### mscore

The **mscore** descriptor provides a score per alignment. It shall be used to represent mapping/alignment score per read generated by genomic sequence reads aligners.

The score shall be expressed using an exponent and fractional part. The number of bits used to represent the exponent and the fractional part are specified in the encoding parameters (see Parameter Set below). Table 12 shows how this is specified in IEEE RFC 754 for a 11-bits exponent and a 52-bits fractional part.

The score of each alignment shall be represented by:

• One sign bit (S)
• 11 bits for the exponent (E)
• 53 bit for the mantissa (M)

**Table 12. Alignment scores are expressed as 64-bit double precision floating point. values**

```
1           11                                              52
+-+----------+-----------------------------------------------+
|S|       Esp        |              Mantissa                 |
+-+----------+---------------------------------------+
6362                          51                                           0
```

If the base (radix) to be used for the calculation of scores is 10, the score is calculated as: score $= -1^s \times 10^E \times M$

### rgroup

The **rgroup** descriptor identifies the read group the Genomic Record belongs to. It is an unsigned 8-bit integer with values going from 0 to **num_groups** - 1. The presence of read groups in an Access Unit is signaled by **num_groups** > 0 in the Parameter Set as defined in the Parameter Set defined below.

### msar

The **msar** (Multiple Segments Alignment Record) descriptor supports spliced reads and alternative secondary alignments which contain indels or soft clips.

**msar** is intended to convey information on:

• a mapped segment length
• a different mapping contiguity (i.e. CIGAR string) for a secondary alignment and/or spliced read

**msar** can is used to represent mismatches, insertions, deletions, strandedness and clipped bases of secondary alignments of an aligned read

### Multiple alignments

The following descriptors are defined for the support of multiple alignments.

### mmap

The **mmap** descriptor is used to signal at how many positions the read or the leftmost read of a pair has been aligned. A Genomic Record containing multiple alignments is associated with one multi-byte **mmap** descriptor. The first two bytes of a **mmap** descriptor represent an unsigned integer N which refers to the read as a single segment (if **spliced_reads_flag** = 0 as defined in this disclosure) or instead to all the segments into which the read has been spliced for the several possible alignments (if **spliced_reads_flag** = 1). The value of N represents the number of values of the **pos** descriptor which are coded for the template in the current record. N is followed by one or more 8-bit unsigned integers $M_i$ as described in this disclosure.

### Multiple alignments strandedness

In case of multiple alignments, the **rcomp** descriptor defined in this disclosure is used to specify the strandedness of each read alignment using the same syntax specified above.

### Scores of multiple alignments

In case of multiple alignments, one **mscore** as defined in this disclosure is assigned to each alignment.

### Multiple alignments without splices

If no splices are present in the Access Unit, **spliced_reads_flag** is unset.

In paired-end sequencing, the **mmap** descriptor is composed by a 16-bit unsigned integer N followed by one or more 8-bit unsigned integers $M_i$, with i assuming values from 1 to the number of complete first (here, the left-most) read alignments. For each first read alignment, spliced or not, $M_i$ is used to signal how many segments are used to align the second read (in this case, without splices, this is equal to the number of alignments), and then how many values of the **pair** descriptor are coded for that alignment of the first read.

The values of $M_i$ shall be used to calculate $P = \sum_{i=1}^{N} M_i$ which indicates the number of alignments of the second read.

A special value of $M_i$ ($M_i = 0$) indicates that the $i^{th}$ alignment of the left-most read is paired with an alignment of the right-most read which is already paired with a $k^{th}$ alignment of the left-most read with k < i (then there is no *new* alignment detected, which is consistent with the equation above).

As an example, in the simplest cases:

1. If there is a single alignment for the leftmost read and two alternative alignments for the rightmost, N assumes the value 1 and $M_1$ assumes the value 2.
2. If two alternative alignments are detected for the leftmost read but only one for the rightmost, N assumes the value 2, $M_1$ assumes the value 1 and $M_2$ assumes the value 0.

When $M_i$ is 0, the associated value of **pair** shall link to an existing second read alignment; a syntax error will be raised otherwise and the alignment considered broken.

*Example*: if the first read has two mapping positions and the second read only one, N is 2, $M_1$ is 1 and $M_2$ is 0 as said earlier. If this is followed by another alternative secondary mapping for the entire template, N assumes the value 3, and $M_3$ assumes the value 1.

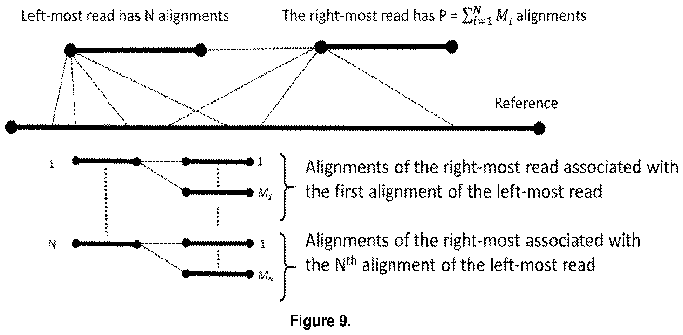

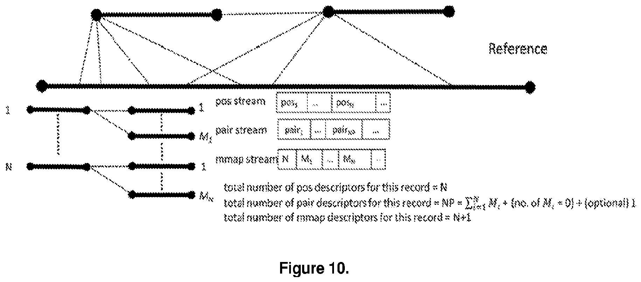

Figure 9 illustrates the meaning of N, P and $M_i$ in case of multiple alignments without splices and Figure 10 shows how the **pos, pair** and **mmap** descriptors are used to encode the multiple alignments information.

With respect to 10 the following applies:

- The right-most read has $P = \sum_{i=1}^{N} M_i$ alignments
- Some values of $M_i$ can be = 0 when the $i^{th}$ alignment of the left-most read is paired with an alignment of the right-most read that is already paired with a $k^{th}$ alignment of the left-most read with k < i
- One reserved value of the pair descriptor can be present to signal alignments belonging to other AUs ranges. If present it's always the first pair descriptor for the current record

### Multiple alignments with splices

If the dataset is encoded with spliced reads, the **msar** descriptor enables representaiton of splices length and strandedness as defined in this disclosure.

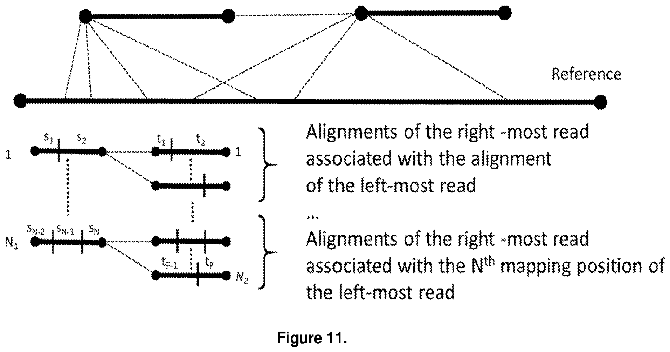

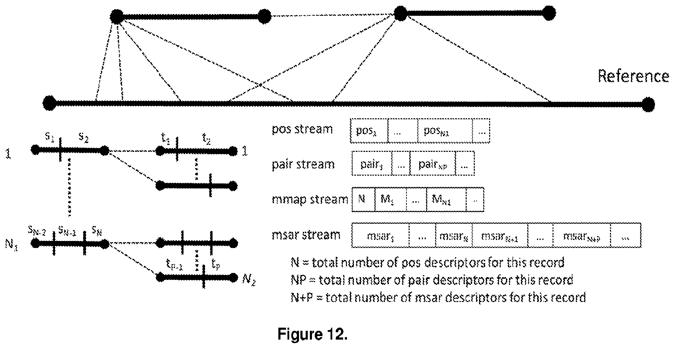

After having decoded the **mmap** and the **msar** descriptors, the decoder knows how many reads or read pairs have been encoded to represent the multiple mappings and how many segments are composing each read or read pair mapping. This is shown in figure 11 and figure 12.

With reference to figure 11 the following applies:

- The left-most read has $N_1$ alignments with N splices ($N_1 \le N$).
- N represents the number of splices present in all alignments of the left-most read and it is encoded as first value of the **mmap** descriptor.

- The right-most read has $P = \sum_{i=1}^{N1} Mi$ splices, where $M_i$ *is* the number of splices of the right-most read which are associated in a pair with the $i^{th}$ alignment of the left-most read ($1 \leq i \leq N_1$). In other words P represents the number of splices of the right-most read and is calculated using the N values following the first value of the **mmap** descriptor.
- $N_1$ and $N_2$ represent the number of alignments of the first and second read and are calculated using the N + P values of the **msar** descriptor.

With reference to figure 12 the following applies:

- The left-most has $N_1$ alignments with N splices ($N_1 \leq N$). If $N_1$ = N AND $N_2$ = P no splices would be present.
- The right-most read has $P = \sum_{i=1}^{N1} Mi$ splices $t_j$ $1 \leq j \leq P$ and $N_2$ ($N_2 \leq P$) alignments.
- The number of **pair** descriptors can be calculated as NP = Max(N1, P) + $M_0$ where

  ◦ $M_0$ is the number of $M_i$ with value 0
  ◦ NP has to be incremented by 1 in case one special pair descriptor indicates the presence of alignments in other AUs.

## Alignment score

The **mscore** descriptor allows signaling the mapping score of an alignment. In single-end sequencing it will have $N_1$ values per template; in paired-end sequencing it will have a value for each alignment of the entire template (number of different alignments of the first read possibly + the number of further second read alignments, i.e. when $M_i$ -1 > 0).

$$\text{Number of scores} = \text{MAX}(N_1, N_2) + M_0$$

where $M_0$ represent the total number of $M_i$ = 0.

The number of scores associated to each alignment is signalled by the encoding parameter **as_depth** as defined in this disclosure.

## Descriptors for multiple alignments without splices

**Table 13. How to calculate the number of descriptors needed to represent multiple alignments in one Genomic Record in case of multiple alignments without splices.**

| Semantic | mmap | mscore | Effect |
|---|---|---|---|
| Read (or paired-end read) with multiple mappings, not spliced | Single read: N Read pair: N, $M_i$ where $1 \leq i \leq N$ | Single read: N values Read pair: MAX(N, $\sum(M_i)$) values + $\sum(M_i = 0)$ Introducing a separator would enable having an arbitrary number of scores. Otherwise a 0 should be used if not present. These are floating point values as defined in this disclosure. | Single read: the read has multiple mappings and it is encoded as a sequence of N consecutive segments belonging to the class with the highest ID. N pos descriptors are used Read pair: the read pair has multiple mappings and it is encoded as a sequence of • N segments for the first read • P =$\sum(M_i)$ pairings to the alignments of the |

(continued)

| Semantic | mmap | mscore | Effect |
|---|---|---|---|
| | | | second read<br>N pos descriptors are used<br>N x P pair descriptors are used with<br>$$NP = \sum_{i=1}^{N} Mi + (\text{no. of } Mi = 0)$$<br>+ (optional) 1<br>The optional pairing descriptor is used when alignments are present on different reference sequences than the one currently encoded<br>N+1 mmap descriptors |
| read (pair) uniquely mapped | 0 | 0 | |

### Descriptors for multiple alignments with splices

Table 14 shows how to calculate the number of descriptors needed to represent multiple alignments in one Genomic Record in case of multiple alignments with splices.

**Table 14. Descriptors used to represent multiple alignments and associated scores.**

| Semantic | mmap | mscore | Effect |
|---|---|---|---|
| Read (or paired-end read) with multiple mappings, with splices | Single read: N<br>Read pair: N, $M_i$ where $1 \le i \le N$ | Single read: $N_1$ values<br>Read pair: $Max(N_1, N_2) + \sum (M_i == 0)$ values<br>$N_1$ and $N_2$ are calculated using the N + P **msar** descriptors<br>$$P = \sum_{i=1}^{N1} Mi$$<br>These are floating point values as defined in this disclosure | Single read:<br>the read has multiple mappings and it is encoded as a sequence of N consecutive segments belonging to the class with the highest ID.<br><br>N **pos** descriptors are used<br>Read pair:<br>the read pair has multiple mappings and it is encoded as a sequence of<br>• N segments for the first read<br>• $P = \sum (M_i)$ pairings to the alignments of the second read<br>N pos descriptors are used |
| read (pair) uniquely mapped | 0 | 0 | |

### Multiple alignments on different sequences

It may happen that the alignment process finds alternative mappings to another reference sequence than the one where the primary mapping is positioned.

For read pairs which are uniquely aligned, a **pair** descriptor shall be used to represent the absolute read positions when there is for example a chimeric alignment with the mate on another chromosome. The **pair** descriptor shall be used to signal the reference and the position of the next record containing further alignments for the same template. The last record (e.g. the third if alternative mappings are coded in three different AUs) shall contain the reference and position of the first record.

In case one or more alignments for the leftmost read in a pair are present on a different reference sequence than the one related to the currently encoded AU, a reserved value of the **pair** descriptor shall be used (not the same as the one used for alignments present to another reference in case of unique alignment). The reserved value shall be followed by the reference sequence identifier and the position of the leftmost alignment among all those contained in the next AU (i.e. the first decoded value of the **pos** descriptor for that record).

### *Multiple alignments with insertions, deletions, unmapped portions*

When an alternative secondary mapping does not preserve the contiguity of the reference region where the sequence is aligned, it may be impossible to reconstruct the exact mapping generated by the aligner because the actual sequence (and then the descriptors related to mismatches such as substitutions or indels) is only coded for the primary alignment. The **msar** descriptor shall be used to represent how secondary alignments map on the reference sequence in case they contain indels and/or soft clips. If **msar** is represented by the special symbol "*" for a secondary alignment, the decoder shall reconstruct the secondary alignment from the primary alignment and the secondary alignment mapping position.

### Raw Data

Raw reads belong to class U only. They are encoded as unmapped reads in aligned datasets. Some of the descriptors defined for reads aligned to an external or internal reference are used to encode raw reads. This is motivated by the fact that raw reads are encoded using reference sequences built from the data to be encoded.

| descriptor_id | Descriptor | Semantics | Comments |
|---|---|---|---|
| 1 | pos | read mapping position | reads positions on the reference are 0-based (signed) delta encoded with respect to the previous read |
| 4 | rcomp | strand information for reads in a template | N bits |
| 8 | flags | used to cover SAM flags and possibly other flags | This is a placeholder to understand the type of flags we need to associate to each template. SAM flags can be a starting point. |
| 5 | mmpos | mismatch position | (unsigned) delta encoded with respect to the previous mismatch |
| 6 | mmtype | type of edit operations: substitutions deletions insertions | fixed alphabet |
| 7 | clips | string of nucleotides with variable length (e.g. soft clips) | ASCII characters + position in the template |
| 11 | ureads | unmapped reads encoded verbatim | ASCII characters |
| 3 | rlen | read lengths | 32-bit integer |
| 12 | rtype | read type | This identifies the subset of descriptors needed to decode the read. |

### *ureads*

The **ureads** descriptor represents verbatim sequence reads as a sequence of ASCII characters belonging to the currently used alphabet.

### *rtype*

The **rtype** descriptor is used to signal the subset of descriptors used to encode one unmapped read or read pair in a Genomic Record as shown in Table 15.

The **rtype** descriptor also enables mixing reference-based and reference-less compression in the same Dataset. In this scenario **rtype** = 0 signals reference based encoded records, while **rtype** > 0 signals the set of descriptors to be used for reference less compression(in this case descriptors refer to the computed reference, when needed).

**Table 15. Semantics of the rtype descriptor.**

| rtype | type of encoded reads | description |
|---|---|---|
| not used | aligned reference based | the entire Dataset is encoded using reference based compression for mapped reads |
| 0 | reference based | the Dataset contains both read (pairs) encoded using reference based compression and reference less compression. Descriptors for this record use the external or embedded reference according to the Class of the AU |
| > 0 | raw and aligned reference less | 1 = class P descriptors used<br>2 = class N descriptors used<br>3= class M descriptors used<br>4 = class I descriptors used<br>5 = class U descriptors used |

**Binarization of descriptors**

In an embodiment, the present invention uses context-adaptive binary arithmetic coding (CABAC) for the compression of the genomic descriptors. CABAC first converts to a binary representation all symbols to be encoded. The process of binarization converts a non-binary-valued symbol (e.g. a mapping position, a mapped read length or a mismatch type) into a binary code prior to arithmetic coding.

The selection of appropriate binarizations adapted to the statistical properties of each descriptor provides better compression ratios than existing formats based on general purpose compressor applied on blocks of heterogeneous elements.

In the following sections these variables are defined:

- *symVal* : non-binary value of the genomic descriptor to be binarized.
- *cLength*: represents the numbers of bits with which the value is binarized.
- *cMax*: is the largest possible value to be binarized. Larger values will be truncated.

While the following binarization tables are calculated for fixed values of these variables, it has to be appreciated that the present principles are not limited to these values, and thus other values can also be used in accordance with the present principles.

Each binarization algorithm used in this disclosure is identified by an identifier as shown in Table 16.

**Table 16. Type of binarizations and respective identifiers.**

| binarization_id | Type of binarization |
|---|---|
| 0 | Binary Coding (BI) |
| 1 | Truncated Unary (TU) |
| 2 | Exponential Golomb (EG) |
| 3 | Signed Exponential Golomb (SEG) |
| 4 | Truncated Exponential Golomb (TEG) |
| 5 | Signed Truncated Exponential Golomb (STEG) |
| 6 | Split Unit-wise Truncated Unary (SUTU) |
| 7 | Signed Split Unit-wise Truncated Unary (SSUTU) |
| 8 | Double Truncated Unary (DTU) |
| 9 | Signed Double Truncated Unary (SDTU) |

### Binary Coding (BI)

This is a standard binary representation whereby each numerical value is coded in its binary representation. The variable cLength - shown in Table 28 when binarization_id = 0 - represents the numbers of bits with which the value will be represented.

### Truncated Unary (TU) Binarization

A TU binary string is the concatenation of *symVal* ones followed by one zero. If *symVal* == *cMax,* the trailing 0-bit is discarded. Table 17 illustrates the bin strings of this truncated unary binarization with *cMax* = 3.

**Table 17. Bin string of the truncated unary binarization with cMax==3.**

| symVal | Binary string | | |
|--------|---------------|---|---|
| 0 | 0 | | |
| 1 | 1 | 0 | |
| 2 | 1 | 1 | 0 |
| 3 | 1 | 1 | 1 |
| binIdx | 0 | 1 | 2 |

The syntax for this binarization process along with arithmetic decoding is described below.

```
decode_cabac_TU(ctxTable, ctxIdx,
cMax) {
    for (binIdx=0; binIdx<cMax; binIdx++) {
        binValue
        if (binValue == 0)
            break
    }
    return binIdx
}
```

**binValue** is the binarized value which can be either 0 or 1.

### Exponential Golomb (EG) Binarization

The parsing process for genomic descriptors binarized using this technique begins with reading the bits starting at the current location in the bitstream up to and including the first non-zero bit, and counting the number of leading bits that are equal to 0.

This process is specified as follows:

```
leadingZeroBits= -1
for( b = 0; !b; leadingZeroBits++ )
    b = read_bits( 1 )
```

The variable *symVal* is then assigned as follows:

$$symVal = 2^{leadingZeroBits} - 1 + read\_bits( leadingZeroBits )$$

where the function call read_bits reads a number of bits from a storage medium equal to the parameter passed as input. The value returned from read_bits( leadingZeroBits ) is interpreted as a binary representation of an unsigned integer with the most significant bit written first.

Table 18 illustrates the structure of the Exp-Golomb code by separating the bit string into "prefix" and "suffix" bits. The "prefix" bits are those bits that are parsed as specified above for the computation of leadingZeroBits, and are shown as either 0 or 1 in the bit string column of Table 18. The "suffix" bits are those bits that are parsed in the computation of symVal and are shown as $x_i$ in Table 18, with i in the range of 0 to leadingZeroBits - 1, inclusive. Each $x_i$ is equal to either 0 or 1.

**Table 18. Binary representations for values of symVal from 0 to 62.**

| Bit string form | Range of symVal |
|---|---|
| 1 | 0 |
| 0 1 $x_0$ | 1..2 |
| 0 0 1 $x_1$ $x_0$ | 3..6 |
| 0 0 0 1 $x_2$ $x_1$ $x_0$ | 7..14 |
| 0 0 0 0 1 $x_3$ $x_2$ $x_1$ $x_0$ | 15..30 |
| 0 0 0 0 0 1 $x_4$ $x_3$ $x_2$ $x_1$ $x_0$ | 31..62 |
| ... | ... |

Table 19 illustrates the explicit assignments of bit strings to symVal values.

**Table 19. Exp-Golomb bit strings and symVal in explicit form.**

| Bit string | symVal |
|---|---|
| 1 | 0 |
| 0 1 0 | 1 |
| 0 1 1 | 2 |
| 0 0 1 0 0 | 3 |
| 0 0 1 0 1 | 4 |
| 0 0 1 1 0 | 5 |
| 0 0 1 1 1 | 6 |
| 0 0 0 1 0 0 0 | 7 |
| 0 0 0 1 0 0 1 | 8 |
| 0 0 0 1 0 1 0 | 9 |
| ... | ... |

Depending on the genomic descriptor, the value of a binarized syntax element is decoded using one of the following methods:

- The value of the decoded genomic descriptor is equal to the *symVal* value corresponding to the binarized descriptor
- The value of the decoded genomic descriptor is calculated by applying signed 0-order Exponential-Golomb decoding as defined for example in https://en.wikipedia.org/wiki/Exponential-Golomb coding with *symVal* as input.

### Signed Exponential Golomb (SEG) Binarization

According to this binarization method the genomic descriptor is associated to the *symVal* by ordering the syntax element by its absolute value in increasing order and representing the positive value for a given absolute value with the lower *symVal*. Table 20 shows the assignment rule.

**Table 20. Assignment of syntax element to symVal for signed Exp-Golomb coded genomic descriptors.**

| symVal | syntax element value |
|---|---|
| 0 | 0 |
| 1 | 1 |
| 2 | -1 |
| 3 | 2 |
| 4 | -2 |
| 5 | 3 |
| 6 | -3 |
| k | $(-1)^{k+1} \mathrm{Ceil}(k \div 2)$ |

### *Truncated Exponential Golomb (TEG) Binarization*

This binarization process requires the use of an additional input parameter *tegParam* which defines how the binarization is calculated.

Output of this process is the TEG binarization of the syntax element.

A TEG bin string is the concatenation of 1 (in case of symVal == 0) or 2 (in case of symVal > 0) types of binarization:

- The truncated unary binarization with cMax = tegParam for the value Min(symVal, tegParam)

- If symVal !=0, the exponential golomb binarization for the value
  Abs(symVal) - tegParam

Table 21 illustrates the bin strings of this Truncated Exponential Golomb binarization with tegParam ==2.

**Table 21. Bin string of the Truncated Exponential Golomb binarization with tegParam=2.**

| symVal | Truncated Unary | | Exponential Golomb | | |
|---|---|---|---|---|---|
| 0 | 0 | - | - | - | - |
| 1 | 1 | 0 | - | - | - |
| 2 | 1 | 1 | 1 | - | - |
| 3 | 1 | 1 | 0 | 1 | 0 |
| 4 | 1 | 1 | 0 | 1 | 1 |
| ... | | | | | |
| binIdx | 0 | 1 | 2 | 3 | 4 |

### *Signed Truncated Exponential Golomb (STEG) Binarization*

This binarization process requires the use of an additional input parameter *stegParam.*

A STEG binary string is the concatenation of 1 (in case of symVal == 0) or 2 (for other cases) binarizations:

1. The truncated exponential golomb binarization for Abs(symVal)
2. If symVal !=0, a one-bit flag equal to 1 (if symVal<0) or equal to 0 (if symVal>0).

Table 22 illustrates the bin strings of this Signed Truncated Exponential Golomb binarization with stegParam = 2.

**Table 22. Binary string of the Signed Truncated Exponential Golomb binarization with stegParam = 2.**

| symVal | Truncated Exponential Golomb | | | | | Sign Flag |
|---|---|---|---|---|---|---|
| | Truncated Unary | | Exponential Golomb | | | |
| ... | | | | | | |
| -4 | 1 | 1 | 0 | 1 | 1 | 1 |
| -3 | 1 | 1 | 0 | 1 | 0 | 1 |
| -2 | 1 | 1 | 1 | - | - | 1 |
| -1 | 1 | 0 | - | - | - | 1 |
| 0 | 0 | - | - | - | - | - |
| 1 | 1 | 0 | - | - | - | 0 |
| 2 | 1 | 1 | 1 | - | - | 0 |
| 3 | 1 | 1 | 0 | 1 | 0 | 0 |
| 4 | 1 | 1 | 0 | 1 | 1 | 0 |
| ... | | | | | | |
| binIdx | 0 | 1 | 2 | 3 | 4 | Max(binIdx)+1 |

### Split Unit-wise Truncated Unary (SUTU) Binarization

This binarization process requires the use of two input parameters *splitUnitSize* and *outputSymSize*. *outputSymSize* must always be a multiple of *splitUnitSize*.

The SUTU binary string is a concatenation of repeated TU binarizations, where each TU binarization is applied to portions of *symVal* which are *splitUnitSize* bits long. In other words, *symVal* is represented by *x* binary string obtained with the TU binarization, where x = outputSymSize/splitUnitSize. The cMax parameter for each binary string is defined as cMax =

(1<<splitUnitSize) - 1.

Table 23 illustrates the binary strings of split unit-wise truncated unary binarizations with splitUnitSize = 2 and outputSymbSize = 8.

**Table 23. Bin string of the Split Unit-wise Truncated Unary binarization with splitUnitSize = 2, outputSymSize = 8.**

| symVal | TU Instance 1 cMax==3 | | | TU Instance 2 cMax==3 | | | TU Instance 3 cMax==3 | | | TU Instance 4 cMax==3 | | |
|---|---|---|---|---|---|---|---|---|---|---|---|---|
| 0 | 0 | - | - | 0 | - | - | 0 | - | - | 0 | - | - |
| 1 | 1 | 0 | - | 0 | - | - | 0 | - | - | 0 | - | - |
| 3 | 1 | 1 | 1 | 0 | - | - | 0 | - | - | 0 | - | - |
| 15 | 1 | 1 | 1 | 1 | 1 | 1 | 0 | - | - | 0 | - | - |
| 31 | 1 | 1 | 1 | 1 | 1 | 1 | 1 | 0 | - | 0 | - | - |
| 63 | 1 | 1 | 1 | 1 | 1 | 1 | 1 | 1 | 1 | 0 | - | - |
| binIdx | 0 | 1 | 2 | 3 | 4 | 5 | 6 | 7 | 8 | 9 | 10 | 11 |

The bitstream syntax for this binarization process is described below.

**Table 24. CABAC decoding process for TU binarization.**

| decode_cabac_SUTU(ctxTable, ctxIdx, splitUnitSize, outputSymSize) { |
|---|

(continued)

```
        output_symb = 0
        cMax = (1<<splitUnitSize) - 1
        for (i=0; i<outputSymSize; i+=splitUnitSize) {
            tmp = decode_cabac_TU(ctxTable, ctxIdx, cMax)
            ctxIdx += cMax
            output_sym |= (tmp<<i)
        }
        return output_sym
}
```

### Signed Split Unit-wise Truncated Unary (SSUTU) Binarization

This binarization process requires the use of two input parameters *splitUnitSize* and *outputSymSize*.
The SSUTU binary string is obtained by an extension of the SUTU binarization process with the sign of *symVal* coded as a separate flag.

- The SUTU binarization for the value Abs(symVal).
- If symVal !=0, a one-bit flag equal to 1 (if symVal<0) or equal to 0 (if symVal>0).

Table 25 illustrates the binary strings of the Signed Split Unit-wise Truncated Unary binarization with splitUnitSize = 2, outputSymbSize = 8.

**Table 25 Bin string of the Signed Split Unit-wise Truncated Unary binarization with splitUnitSize = 2, outputSymSize = 8.**

| symVal | TU Instance 1 cMax==3 | | | TU Instance 2 cMax==3 | | | TU Instance 3 cMax==3 | | | TU Instance 4 cMax==3 | | | Sign |
|---|---|---|---|---|---|---|---|---|---|---|---|---|---|
| -63 | 1 | 1 | 1 | 1 | 1 | 1 | 1 | 1 | 1 | 0 | - | - | 1 |
| -31 | 1 | 1 | 1 | 1 | 1 | 1 | 1 | 0 | - | 0 | - | - | 1 |
| -15 | 1 | 1 | 1 | 1 | 1 | 1 | 0 | - | - | 0 | - | - | 1 |
| -3 | 1 | 1 | 1 | 0 | - | - | 0 | - | - | 0 | - | - | 1 |
| -1 | 1 | 0 | - | 0 | - | - | 0 | - | - | 0 | - | - | 1 |
| 0 | 0 | - | - | 0 | - | - | 0 | - | - | 0 | - | - | - |
| 1 | 1 | 0 | - | 0 | - | - | 0 | - | - | 0 | - | - | 0 |
| 3 | 1 | 1 | 1 | 0 | - | - | 0 | - | - | 0 | - | - | 0 |
| 15 | 1 | 1 | 1 | 1 | 1 | 1 | 0 | - | - | 0 | - | - | 0 |
| 31 | 1 | 1 | 1 | 1 | 1 | 1 | 1 | 0 | - | 0 | - | - | 0 |
| 63 | 1 | 1 | 1 | 1 | 1 | 1 | 1 | 1 | 1 | 0 | - | - | 0 |
| binIdx | 0 | 1 | 2 | 3 | 4 | 5 | 6 | 7 | 8 | 9 | 10 | 11 | 12 |

The syntax for this binarization process is described below.

```
decode_cabac_SSUTU(ctxTable, ctxIdx, splitUnitSize, outputSymSize) {
    output_sym = decode_cabac_SUTU(ctxTable, ctxIdx, splitUnitSize,
            outputSymSize)
    if(output_sym > 0) {
        ctxIdx += ((1<<splitUnitSize) - 1) * (outputSymSize / splitUnitSize)
    sign_flag
```

(continued)

```
        if(sign_flag == 1)
            output_sym = -output_sym }
    }
        return output_sym
}
```

**sign_flag** represents the cabac decoding of a bit on context variable identified by ctxIdx. **decode_cabac_SUTU()** represents the cabac decoding process for the SUTU binarization.

### Double Truncated Unary (DTU) Binarization

This binarization process requires the use of two input parameters *splitUnitSize* and *outputSymSize*.

The DTU binary string is a concatenation of two binarizations, namely the TU binarization and the SUTU binarization. The parameter *cMax* is used for the TU binarization, and parameters splitUnitSize and outputSymSize are used for the SUTU binarization (where its *cMax* is derived internally).

- The first instance of the TU binarization for the value Min(Abs(symVal), cMax).
- If Abs(symVal) > cMax, the second instance of SUTU binarization for the value Abs(symVal) - cMax.

Table 26 illustrates the binary strings of the Double Truncated Unary binarization with cMax = 1, splitUnitSize = 2, outputSymSize = 8.

**Table 26 Bin string of the Double Truncated Unary binarization with cMax = 1, splitUnitSize = 2, outputSymSize = 8.**

| symVal | TU Instance cMax=1 | SUTU Instance: splitUnitSize = 2, outputUnitSize = 8 | | | | | | | | | | | |
|---|---|---|---|---|---|---|---|---|---|---|---|---|---|
| | | TU Instance 1 cMax=3 | | | TU Instance 2 cMax=3 | | | TU Instance 3 cMax=3 | | | TU Instance 4 cMax=3 | | |
| 0 | 0 | - | - | - | - | - | - | - | - | - | - | - | - |
| 1 | 1 | - | - | - | - | - | - | - | - | - | - | - | - |
| 3 | 1 | 1 | 1 | 0 | 0 | - | - | 0 | - | - | 0 | - | - |
| 15 | 1 | 1 | 1 | 0 | 1 | 1 | 1 | 0 | - | - | 0 | - | - |
| 31 | 1 | 1 | 1 | 0 | 1 | 1 | 1 | 1 | 0 | - | 0 | - | - |
| 63 | 1 | 1 | 1 | 0 | 1 | 1 | 1 | 1 | 1 | 1 | 0 | - | - |
| binIdx | 0 | 1 | 2 | 3 | 4 | 5 | 6 | 7 | 8 | 9 | 10 | 11 | 12 |

The binarization process is described below.

```
decode_cabac_DTU(ctxTable, ctxIdx, cMax, splitUnitSize, outputSymSize) {
    output_sym = 0
  if(cMax > 0) {
        output_sym = decode_cabac_TU(ctxTable, ctxIdx, cMax)
    if(output_sym > cMax) {
    output_sym += decode_cabac_SUTU(ctxTable, ctxIdx+cMax,
            splitUnitSize, outputSymSize)
        }
    } else
    output_sym = decode_cabac_SUTU(ctxTable, ctxIdx,
            splitUnitSize, outputSymSize)
  return output_sym
```

(continued)

|  |
|---|
| } |

**decode_cabac_TU()** represents the cabac decoding process for TU binarization.

**decode_cabac_SUTU()** represents the cabac decoding process for SUTU binarization.

### *Signed Double Truncated Unary (SDTU) Binarization*

This binarization process requires the use of two additional input parameters *splitUnitSize* and *outputSymSize.*

The SDTU binary string is obtained by an extension of the DTU binarization process with the sign of *symVal* coded as a flag.

- The DTU binarization for the value Abs(symVal).
- If symVal !=0, a one-bit flag equal to 1 (if symVal<0) or equal to 0 (if symVal>0).

Table 27 illustrates the bin strings of double truncated unary binarization with with cMax = 1, splitUnitSize = 2, outputSymSize = 8.

**Table 27 Bin string of the Signed Double Truncated Unary binarization with with cMax = 1, splitUnitSize = 2, outputSymSize = 8.**

| symVal | TU Instance cMax=1 | SUTU Instance: splitUnitSize = 2, outputUnitSize = 8 | | | | | | | | | | | | Sign |
|---|---|---|---|---|---|---|---|---|---|---|---|---|---|---|
| | | TU Instance 1 cMax=3 | | | TU Instance 2 cMax=3 | | | TU Instance 3 cMax=3 | | | TU Instance 4 cMax=3 | | | |
| -63 | 1 | 1 | 1 | 0 | 1 | 1 | 1 | 1 | 1 | 1 | 0 | - | - | 1 |
| -31 | 1 | 1 | 1 | 0 | 1 | 1 | 1 | 1 | 0 | - | 0 | - | - | 1 |
| -15 | 1 | 1 | 1 | 0 | 1 | 1 | 1 | 0 | - | - | 0 | - | - | 1 |
| -3 | 1 | 1 | 1 | 0 | 0 | - | - | 0 | - | - | 0 | - | - | 1 |
| -1 | 1 | - | - | - | - | - | - | - | - | - | - | - | - | 1 |
| 0 | 0 | - | - | - | - | - | - | - | - | - | - | - | - | - |
| 1 | 1 | - | - | - | - | - | - | - | - | - | - | - | - | 0 |
| 3 | 1 | 1 | 1 | 0 | 0 | - | - | 0 | - | - | 0 | - | - | 0 |
| 15 | 1 | 1 | 1 | 0 | 1 | 1 | 1 | 0 | - | - | 0 | - | - | 0 |
| 31 | 1 | 1 | 1 | 0 | 1 | 1 | 1 | 1 | 0 | - | 0 | - | - | 0 |
| 63 | 1 | 1 | 1 | 0 | 1 | 1 | 1 | 1 | 1 | 1 | 0 | - | - | 0 |
| binIdx | 0 | 1 | 2 | 3 | 4 | 5 | 6 | 7 | 8 | 9 | 10 | 11 | 12 | 13 |

The syntax for this binarization process is described below.

```
decode_cabac_SDTU(ctxTable, ctxIdx, cMax, splitUnitSize, outputSymSize)
{
    output_sym = decode_cabac_DTU(ctxTable, ctxIdx, cMax,
                splitUnitSize, outputSymSize)
    if(output_sym > 0) {
    ctxIdx += cMax + ((1<<splitUnitSize) - 1) *
                        (outputSymSize / splitUnitSize)
        sign_flag
    if(sign_flag == 1)
            output_sym = -output_sym
    }
```

(continued)

```
    return output_sym
}
```

**sign_flag** represents the cabac decoding of a bit on context variable identified by ctxIdx.

**decode_cabac_DTU()** represents the cabac decoding with DTU binarization.

### Binarization parameters

Each binarization algorithm introduced in the previous sections requires configuration parameters at the encoding and decoding ends. In an embodiment, said configuration parameters are encapsulated in a data structure described in Table 28. Each binarization algorithm is identified by an identifier as listed in Table 16.

**Table 28. Binarization parameters structure**

| Binarization ID | parameters |
|---|---|
| 0 | cLength |
| 1 | cMax |
| 2 | - |
| 3 | - |
| 4 | tegParam |
| 5 | stegParam |
| 6 | splitUnitSize, outputSymSize |
| 7 | splitUnitSize, outputSymSize |
| 8 | cMax, splitUnitSize, outputSymSize |
| 9 | cMaxsplitUnitSize, outputSymSize |

In Table 28 the following semantics applies:

**cMax** represents the largest value to be binarized. Larger values will be truncated.
**cLength** represents the numbers of bits with which the value is binarized.
**tegParam** represents the tegParam variable defined in this disclosure for TEG binarization.
**stegParam** represents the stegParam variable defined in this disclosure for STEG binarization.
**splitUnitSize** represents the splitUnitSize variable defined in this disclosure for SUTU, SSUTU and DTU binarizations.
**outputSymSize** represents the outputSymSize variable defined in this disclosure for SUTU, SSUTU DTU and SDTU binarizations.

### Evidence of technical advantage of present invention

By applying the indicated CABAC binarization to the respective genomic descriptors as indicated in Table 29, the compression performance reported in * no additional information is necessary since it is already available in the compressed representation according to the principles of this disclosure.

Table 30 can be obtained. The improvement in compression performance of the method described in this disclosure can be appreciated by comparison with the corresponding file sizes of BAM and CRAM approaches and one of the best compressors in literature known as DeeZ (see Numanagic, I., et al "Comparison of high-throughput sequencing data compression tools", Nature Methods (ISSN: 1548-7091), vol. 13, p. 1005-1008 London: Nature Publishing Group, 2016). It has to be appreciated that the DeeZ, BAM and CRAM compression performance are calculated by adding the size of the compressed reference genome used for alignment to the sizes of the compressed genome sequence data. According to the principles of the present disclosure, the reference genome is embedded in the compressed file. In today practice said compressed reference genome is a PASTA (ASCII text) file compressed using general purpose compressors such as GZIP, LZMA, Bzip2. In the proposed comparison the reference genome hs37d5.fa was compressed using the **xz** Linux command with the option of maximum compression (-9).

### Binarization applied to descriptors

Table 30 shows the binarization applied to the genomic descriptors defined in this disclosure. When a concatenation of several binarizations is indicated, the different binarizations are applied to the different elements composing each descriptor as defined in this disclosure.

**Table 29. Binarizations associated to each genomic descriptor.**

| descriptor_id | short name | binarization_id |
|---|---|---|
| 1 | pos | 8 (aligned reads) 9 (raw reads) |
| 2 | pair | 0, 6, 6, 6 |
| 3 | rlen | 6 |
| 4 | rcomp | 1 |
| 5 | mmpos | 0, 6 |
| 6 | mmtype | 0, 0, 1, 1 |
| 7 | clips | 5, 1, 3, 0 |
| 8 | flags | 0 |
| 9 | mmap | 6 |
| 10 | msar | 3 |
| 11 | ureads | 1 |
| 12 | rtype | 1 |
| 13 | rftp | 0, 6 |
| 14 | rftt | 0, 0, 1, 1 |
| 15 | rgroup | 1 |
| 16 | mscore | 1 |

### rftp and rftt

An example of binarization of rftp and rftt is provided in this section and illustrated in figure 10. The descriptors associated to five mismatches between a contig and a reference genome used for alignment are shown below:

| rftp | 5 | 7 | 12 | 13 | 15 |
|---|---|---|---|---|---|
| rftt | C | T | T | C | A |

Each nucleotide symbol is associated to an integer code:

| Nucleotide | code |
|---|---|
| A | 0 |
| C | 1 |
| G | 2 |
| T | 3 |
| N | 4 |

After transformation the values become:

| rftp | 5 | 2 | 5 | 1 | 2 |
|------|---|---|---|---|---|
| rftt | 1 | 3 | 3 | 1 | 0 |

The binarized values for **rftp** are calculates as follows:

1. The terminator value can be binarized as 0 or 1. Here for this example we select 0.
2. If terminator = 0 then binarization no. 6 with splitUnitSize = 4, outputSymbolSize = 12 is used and the following binary strings are associated to the values of rftp

    a. 5= 11110
    b. 2 = 110
    c. 5= 11110
    d. 1 = 10
    e. 2 = 110

The binarized values for **rftt** are calculates as follows:
1. Knowing the nucleotide present in the reference genome, remove the corresponding symbol from the possible symbols to be encoded. I.e. for the first mismatch of the example, if the corresponding symbol in the reference is a 'G', the space of possible symbols to be encoded is 0, 1, 3, 4.
2. The frequencies of symbols of the mismatches types on the data to be encoded are measured and indexed from 0 to 3. Index 0 is affected to the most frequent mismatch and index 3 is affected to the less frequent mismatch. In this example an indexing could be: { 0 => 3, 1=> 0, 2=>4, 3=>1}
3. In the given example the five mismatches would be binarized using the TU binarization as:

| Symbol | index | TU binarization with cMax = 3 |
|--------|-------|-------------------------------|
| 1 | 3 | 111 |
| 3 | 0 | 0 |
| 3 | 0 | 0 |
| 1 | 3 | 0 |
| 0 | 1 | 10 |

With the binarization approach shown above the following compression results are achieved:

**Table 30. Compression performance with respect to state of the art solutions (sizes in bytes).**

| Compressor | BAM | CRAM | Deez | Proposed method |
|------------|-----|------|------|-----------------|
| 9827_2#49.bam (ERR317482) - Low coverage | 4,755,859,110 | 3,124,448,497 | 2,592,665,720 | 2,164,362,407 |
| Reference genome hs37d5.fa | 707,712,316 | 707,712,316 | 707,712,316 | N/A* |
| Total | 5,463,571,426 | 3,832,160,813 | 3,300,378,036 | 2,164,362,407 |
| NA12878_S1.bam - High Coverage | 117,653,446,187 | 64,565,636,391 | 64,334,196,408 | 47,759,141,388 |
| Reference genome hs37d5.fa | 707,712,316 | 707,712,316 | 707,712,316 | N/A* |
| Total | 118,361,158,503 | 65,273,348,707 | 65,041,908,724 | 47,759,141,388 |
| * no additional information is necessary since it is already available in the compressed representation according to the principles of this disclosure. | | | | |

**Coding parameters**

In an embodiment, the parameters needed to encode and decode each Access Unit are encapsulated in a data structure named Parameter Set as defined in Table 31.

**Table 31. Coding parameters for genomic descriptors**

| Parameter Set | | |
|---|---|---|
| Parameter name | Cardinality | Description |
| dataset type | 1 | type of data encoded in Access Units referring to this encoding parameters. |
| reads length | 1 | length in nucleotides of sequence reads in case of constant reads length. The value 0 indicates the presence of variable reads length (conveyed by the **rlen** syntax element as defined in this disclosure). |
| QV depth | 1 | number of Quality Values associated to each coded nucleotide. 0 means that no Quality Values are encoded. |
| alignment score depth | 1 | number of alignments scores associated to each coded alignments. 0 means that no alignment scores are encoded. |
| terminator size | 1 | represents the size in bytes minus one (e.g. 0 = 1 byte) of the terminator symbol used for the **mmpos** descriptor defined in **Error! Reference source not found.** |
| terminator value | 1 | represents the value of the terminator symbol used for the **mmpos** descriptor defined in Table 1. |
| number of classes | 1 | number of data classes encoded in all Access Units referring to these encoding parameters. |
| class ID | number of classes | identifier associated to one of the data class defined in this disclosure (P, N, M, I, HM, U). |
| number of descriptors | 1 | total number of descriptors contained in Access Units referring to these configuration parameters |
| coding mode ID | number of descriptors | one of the coding modes defined in this disclosure |
| number of groups | 1 | number of different values of the **rgroup** descriptor listed in table 1 present in all Access Units referring to the current encoding parameters. |
| group name | number of groups | null-terminated string identifier of a read group. |
| multiple alignments flag | 1 | flag signaling the presence of multiple alignments in the Access Unit. When set to 0 no multiple alignments are present |
| spliced reads flag | 1 | flag signaling the presence of spliced reads in the Access Unit. When set to 0 no spliced reads are present. |
| multiple signature base | 1 | flag signaling the use of multiple signatures in an Access Unit containing unmapped sequence reads (Class U). |
| signature size | 1 | size in bits of each integer representing an encoded signature. |
| score exponent | 1 | number of bits used to encode the exponent part of the multiple alignments score encoded in the **mscore** descriptor. As specified in IEEE RFC 754 the value can go from 0 to 11. The **mscore** descriptor is defined in table 1. |

(continued)

| Parameter Set | | |
|---|---|---|
| Parameter name | Cardinality | Description |
| score fractional | 1 | number of bits used to encode the fractional part of the multiple alignments score encoded in the **mscore** descriptor. As specified in IEEE RFC 754 the value can go from 0 to 52. The **mscore** descriptor is defined in table 1. |
| contig buffer size | 1 | size in bytes of the buffer used to build the contig 102 in figure 1. |
| contig buffer count | 1 | number of reads used to build the contig 102 in figure 1. |

**Encoding apparatus**

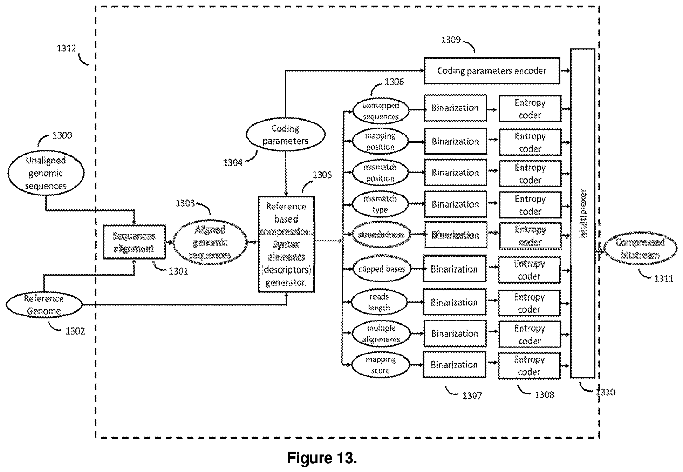

Figure 13 shows an encoding apparatus according to the principles of this invention. The encoding apparatus receives as input a reference genome 1302 and unaligned genomic sequences 1300, for example produced by a genome sequencing apparatus. Genome sequencing apparatus are known in the art, like the Illumina HiSeq 2500, the Thermo-Fisher Ion Torrent devices or the Oxford Nanopore MinION. The unaligned sequence data 1300 are fed to a reads alignment unit 1301, which maps the sequences on a reference genome 1302. The aligned genomic sequences 303 are then fed to a reference based compressor 1305 which generates genomic descriptors 1306 representing both mapped and unmapped genomic sequences. The genomic descriptors 1306 generated by the reference based compressor 1305 are first binarized by several binarization units 1307 and then entropy coded by several entropy coders 1308. The entropy coded genomic descriptors are then fed to a multiplexing apparatus 1310 to build one or more Access Unit composing a compressed bitstream 1311. The multiplexed bitstream contains as well coding parameters structures 1304 built by a coding parameters encoder 1309. Each Access Unit contains entropy coded descriptors representing alignment information and sequence reads belonging to one class of data as defined in this disclosure.

**Decoding apparatus**

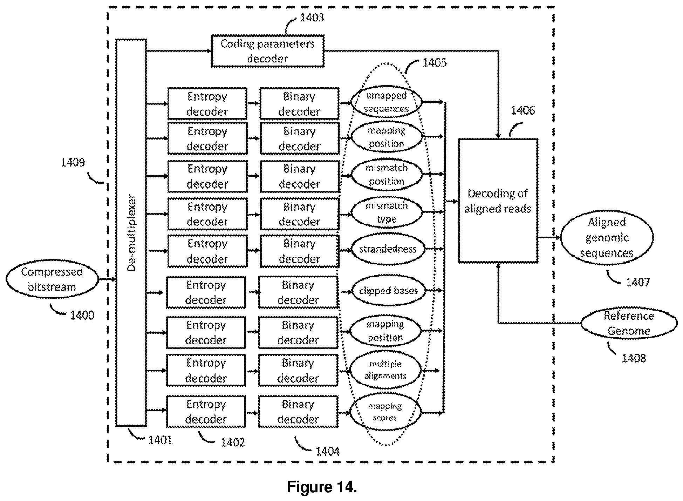

Figure 14 shows a decoding apparatus according to the principles of this disclosure. A demultiplexing unit 1401 receives a multiplexed bitstream 1400 from a network or a storage element and extracts the entropy coded payload of the Access Units composing said bitstream. Entropy decoders 1402 receive the extracted payloads and decode the different types of genomic descriptors into their binary representations. Said binary representations are then fed to several binary decoders 1404 which generate genomic descriptors 1405. A coding parameter decoder 1403 receives coding parameters multiplexed with the genomic information and feds them to the decoding unit 1406. Genomic descriptors 1405 representing genomic sequence reads are fed to a sequence reads reconstruction unit 1406 which reconstructs the aligned genomic sequences 1407 using an available reference genome 1408.

The inventive techniques herewith disclosed may be implemented in hardware, software, firmware or any combination thereof. When implemented in software, these may be stored on a computer medium and executed by a hardware processing unit. The hardware processing unit may comprise one or more processors, digital signal processors, general purpose microprocessors, application specific integrated circuits or other discrete logic circuitry.

The techniques of this disclosure may be implemented in a variety of devices or apparatuses, including mobile phones, desktop computers, servers, tablets and similar devices.

**Claims**

1. A computer-implemented method for encoding genome sequence data, said genome sequence data comprising reads of sequences of nucleotides, said method comprising the steps of:

   aligning said reads to one or more reference sequences thereby creating aligned reads,
   classifying said aligned reads into different classes according to specified matching rules with said one or more reference sequences, wherein said classifying comprises:

      - classifying one or more of said aligned reads into a first class (Class P) when a region in the reference sequence is found to match the aligned read without any mismatch;

- classifying one or more of said aligned reads into a second class (Class N) when a region in the reference sequence is found to match the aligned read with a type and a number of mismatches determined only by the number of positions in which a sequencing machine generating the read was not able to call any base;
- classifying one or more of said aligned reads into a third class (Class M) when a region in the reference sequence is found to match the aligned read with a type and a number of mismatches determined only by the number of positions in which the sequencing machine generating the read was not able to call any base, plus the number of mismatches in which a different nucleotide than the one present in the reference sequence has been called;
- classifying one or more of said aligned reads into a fourth class (Class I) constituted by aligned reads presenting at least one mismatch of any type among insertion, deletion and clipped plus, if present, any mismatches type belonging to the second class (Class N) or third class (Class M), wherein insertions are constituted by an additional sequence of one or more nucleotides not present in the reference sequence, but present in the aligned read, wherein deletions constitute missing nucleotides in the aligned read with respect to the reference sequence, and wherein clipped comprises soft clipped nucleotides, which represent an inserted nucleotide sequence at the edges of the aligned read which are not matching the reference sequence but are kept in the aligned read, and hard clipped nucleotides, which are discarded from the aligned read;
- classifying one or more of said aligned reads into a fifth class (Class U) when no matching according to any of the first to fourth class is found;

thereby creating classes of aligned reads, represented by groups of genomic descriptors that univocally represent genome sequence reads and that are organized in blocks with homogeneous statistical properties, wherein the descriptors comprise, for the first class (Class P):

- the mapping position of a read on the reference sequence (pos),
- the DNA or RNA strand the read was mapped on (rcomp), and
- mapping flags for enabling the aligner to further specify the result of the mapping process;

wherein the descriptors further comprise, for the second class (Class N):

- the position of mismatches in aligned reads with respect to reference sequences (mmpos);

wherein the descriptors further comprise, for the third class (Class M):

- the types of mismatches with respect to reference sequences at the associated positions (mmtype);

wherein the descriptors further comprise, for the fourth class (Class I):

- a descriptor signaling soft or hard clipped nucleotides (clips);

wherein the descriptors further comprise, for the fifth class (Class U):

- verbatim sequence reads of bases that cannot be mapped on any available reference sequence (ureads);

encoding said classified aligned reads represented by blocks of descriptors with homogeneous statistical properties as a multiplicity of blocks of syntax elements, and
structuring said blocks of syntax elements with header information thereby creating successive Access Units, wherein said encoding further comprises binarizing and entropy coding said genomic descriptors, said binarizing and entropy coding being performed according to each descriptor specific statistical properties,
wherein said binarizing and entropy coding genomic descriptors is such that the binarization for at least one descriptor of said descriptors is different from the binarization for at least one other descriptor of said descriptors.

**2.** The method of claim 1, further comprising coding one or more of the following descriptors:

rlen descriptor for signaling the length of each encoded sequence read;
mmap for signaling the multiple mapping positions that are associated to a single read or read pair by the mapping procedure;

msar for signaling the identification of the existence of spliced reads (i.e. reads that when split in chunks find mapping positions with higher degrees of matching accuracy than when they are mapped as single contiguous read mapped on a single position on a reference sequence).

mscore descriptor to signal a mapping/alignment score per read as generated by genomic sequence reads aligners;

pair descriptor to signal, in case of paired end reads, how the reads are paired;

rtype descriptor used to signal the subset of descriptors used to encode sequence reads that cannot be mapped at any position of the reference sequence with specified degrees of matching accuracy;

rgroup descriptor to signal to which read group the read belongs to;

rftp for signaling the position of mismatches between a contig and a reference sequence, wherein positions of mismatches are terminated by a special terminator character,

rftt for signaling the type of mismatches between a contig and a reference sequence.

3. The method of claim 1 or 2, wherein said descriptors are binarized as follows:

said pos descriptor is binarized using a double truncated unary code or a single double truncated unary code;

said rcomp descriptor is binarized using a truncated unary code;

said mapping flags descriptors are binarized using binary coding;

said mmpos descriptor for signaling the position of mismatches in aligned reads with respect to reference sequences is binarized using a split unit wise truncated unary code;

said mmtype descriptor for signaling the types of mismatches with respect to reference sequences at the associated positions is binarized using a truncated unary code;

said clips descriptor for signaling soft or hard clipped nucleotides is binarized using a concatenation of Signed Truncated Exponential Golomb, Truncated Unary, Signed Exponential Golomb and Binary Codes.

4. The method of claim 2, or claim 3 when depending on claim 2, wherein one or more of said descriptors are binarized as follows:

said rlen descriptor signaling the length of each encoded sequence read is binarized using a Split Unit-wise Truncated Unary code;

said mmap descriptor for signaling the multiple mapping positions that are associated to a single read or read pair by the mapping procedure is binarized using a Split Unit-wise Truncated Unary code;

said msar descriptor for signaling the identification of the existence of spliced reads is binarized using a Signed Exponential Golomb code;

said mscore descriptor to signal a mapping/alignment score per read as generated by genomic sequence reads aligners is binarized using a Truncated Unary code;

said pair descriptor to signal, in case of paired end reads, how the reads are paired is binarized using a concatenation of Binary Coding and Split Unit-wise Truncated Unary code;

said ureads descriptor to signal reads which could not be aligned at any position of the reference sequence is binarized using a Truncated Unary code;

said rtype descriptor used to signal the subset of descriptors used to encode sequence reads that cannot be mapped at any position of the reference sequence with specified degrees of matching accuracy is binarized using a Truncated Unary code;

said rgroup descriptor to signal to which read group the read belongs to is binarized using a Truncated Unary code;

said rftp descriptor for signaling the position of mismatches between a contig and a reference sequence is binarized using a concatenation of Binary Coding and Split Unit-wise Truncated Unary code; and

said rftt descriptor for signaling the type of mismatches between a contig and a reference sequence is binarized using a concatenation of Binary Coding and Truncated Unary code.

5. The method of any one of the preceding claims, wherein said descriptors are coded by inserting configuration parameters in a syntax header.

6. The method of claim 5, wherein said configuration parameters are updated by creating updated syntax headers to be added to the encoded genomic file.

7. The method of claim 5 or 6, wherein said configuration parameters comprise one or more of the following:

a dataset type for signaling the type of data encoded in Access Units referring to these configuration parameters;

a read length for signaling the length in nucleotides of sequence reads in case of constant read length;

a quality values depth parameter for signaling the number of Quality Values associated to each coded nucleotide;

an alignment score depth for signaling the number of alignments scores associated to each coded alignments;

a terminator size for signaling the size in bytes of the terminator symbol used for the mmpos descriptor;

a terminator value for signaling the value of the terminator symbol used for the mmpos descriptor; the number of classes for signaling the number of data classes encoded in all Access Units referring to said configuration parameters; class identifiers to signal the identifiers associated to each data class;

the number of descriptors for signaling the total number of descriptors contained in Access Units referring to said configuration parameters;

coding mode identifiers for the different coding modes;

a number of groups parameter for signaling the number of different values of the rgroup descriptor present in all Access Units referring to the current configuration parameters;

one or more group narre parameters for signaling one or more read group identifiers;

a multiple alignments flag for signaling the presence of multiple alignments in the Access Unit; and

a spliced reads flag for signaling the presence of spliced reads in the Access Unit.

**8.** The method of any one of claims 5 to 7, wherein said configuration parameters further comprise a multiple signature base flag for signaling the use of multiple signatures in an Access Unit containing unmapped sequence reads (Class U), preferably wherein said configuration parameters further comprise a signature size for signaling the size in bits of each integer representing an encoded signature.

**9.** The method of any one of the preceding claims, wherein said entropy coding of said descriptors is performed using a multiplicity of entropy coders.

**10.** The method of claim 9, wherein said binarizing and entropy coding is such that the entropy coding for at least one descriptor of said descriptors is different from the entropy coding for at least one other descriptor of said descriptors.

**11.** A computer-implemented method for decoding encoded genomic data, wherein said genome sequence data comprises reads of sequences of nucleotides, said method comprising the steps of:

parsing Access Units containing said encoded genomic data to extract multiple blocks of genomic descriptors by employing header information; and

decoding said multiplicity of blocks;

wherein said decoding of multiplicity of blocks comprises entropy decoding and de-binarizing genomic descriptors arranged in blocks of homogeneous data to carry out an extraction of aligned reads according to specific matching rules defining their classification with respect to one or more reference sequences, wherein:

- when one or more of said aligned reads are classified into a first class (Class P), said extraction is carried out based on a region in the reference sequence matching the one or more aligned reads without any mismatch;

- when one or more of said aligned reads are classified into a second class (Class N), said extraction is carried out based on a region in the reference sequence matching the one or more aligned reads with a type and a number of mismatches determined only by the number of positions in which a sequencing machine generating the read was not able to call any base;

- when one or more of said aligned reads are classified into a third class (Class M), said extraction is carried out based on a region in the reference sequence matching the one or more aligned reads with a type and a number of mismatches determined only by the number of positions in which the sequencing machine generating the read was not able to call any base, plus the number of mismatches in which a different nucleotide than the one present in the reference sequence has been called;

- when one or more of said aligned reads are classified into a fourth class (Class I), said extraction is carried out based on said one or more aligned reads presenting at least one mismatch of any type among insertion, deletion and clipped plus, if present, any mismatches type belonging to the second class (Class N) or third class (Class M), wherein insertions are constituted by an additional sequence of one or more nucleotides not present in the reference sequence, but present in the aligned read, wherein deletions constitute missing nucleotides in the aligned read with respect to the reference sequence, and wherein clipped comprises soft clipped nucleotides, which represent an inserted nucleotide sequence at the edges of the aligned read which are not matching the reference sequence but are kept in the aligned read, and hard clipped nucleotides, which are discarded from the aligned read;

- when one or more of said aligned reads are classified into a fifth class (Class U), said extraction is carried out based on the fact that no matching according to any of the first to fourth class is present;

wherein the descriptors comprise, for the first class (Class P):

- the mapping position of a read on the reference sequence (pos),
- the DNA or RNA strand the read was mapped on (rcomp), and
- mapping flags for enabling the aligner to further specify the result of the mapping process;

wherein the descriptors further comprise, for the second class (Class N):

- the position of mismatches in aligned reads with respect to reference sequences (mmpos);

wherein the descriptors further comprise, for the third class (Class M):

- the types of mismatches with respect to reference sequences at the associated positions (mmtype);

wherein the descriptors further comprise, for the fourth class (Class I):

- a descriptor signaling soft or hard clipped nucleotides (clips);

wherein the descriptors further comprise, for the fifth class (Class U):

- verbatim sequence reads of bases that cannot be mapped on any available reference sequence (ureads);

wherein said entropy decoding and de-binarizing genomic descriptors comprises, for at least one descriptor of said descriptors, employing a de-binarization that is different from a de-binarization used for at least one other descriptor of said descriptors.

12. The method of claim 11, further comprising decoding the one or more of the following descriptors:

rlen descriptor for signaling the length of each encoded sequence read;
mmap for signaling the multiple mapping positions that are associated to a single read or read pair by the mapping procedure;
msar for signaling the identification of the existence of spliced reads, i.e. reads that when split in chunks find mapping positions with higher degrees of matching accuracy than when they are mapped as single contiguous read mapped on a single position on a reference sequence;
mscore descriptor to signal a mapping/alignment score per read as generated by genomic sequence reads aligners;
pair descriptor to signal, in case of paired end reads, how the reads are paired;
ureads descriptor to signal reads which could not be aligned at any position of the reference sequence;
rtype descriptor used to signal the subset of descriptors used to encode sequence reads that cannot be mapped at any position of the reference sequence with specified degrees of matching accuracy;
rgroup descriptor to signal to which read group the read belongs to;
rftp for signaling the position of mismatches between a contig and a reference sequence, wherein positions of mismatches are terminated by a special terminator character;
rftt for signaling the type of mismatches between a contig and a reference sequence.

13. The method of claim 11 or 12, wherein said descriptors are decoded by extracting configuration parameters from a syntax header.

14. The method of claim 13, wherein said configuration parameters comprise one or more of the following:

a dataset type for signaling the type of data encoded in Access Units referring to these configuration parameters;
a read length for signaling the length in nucleotides of sequence reads in case of constant read length;
a quality values depth parameter for signaling the number of Quality Values associated to each coded nucleotide;
an alignment score depth for signaling the number of alignments scores associated to each coded alignment;
a terminator size for signaling the size in bytes of the terminator symbol used for the mmpos descriptor; a terminator value for signaling the value of the terminator symbol used for the mmpos descriptor;
the number of classes for signaling the number of data classes encoded in all Access Units referring to said configuration parameters;
class identifiers to signal the identifiers associated to each data class;
the number of descriptors for signaling the total number of descriptors contained in Access Units referring to said configuration parameters;
coding mode identifiers for signaling the coding modes defined in this disclosure;
a number of groups parameter for signaling the number of different values of the rgroup descriptor present in all Access Units referring to the current configuration parameters;
one or more group name parameters for signaling one or more read group identifiers;
a multiple alignments flag for signaling the presence of multiple alignments in the Access Unit; and
a spliced reads flag for signaling the presence of spliced reads in the Access Unit, with the proviso that when set to O no spliced reads are present.

15. The method of claim 13, or claim 14 when depending on claim 13, wherein said configuration parameters further comprise a multiple signature base flag for signaling the use of multiple signatures in an Access Unit containing unmapped sequence reads (Class U); and preferably said configuration parameters further comprise a signature size for signaling the size in bits of each integer representing an encoded signature.

16. The method of anyone of claims 11 to 15, wherein said entropy decoding and de-binarizing genomic descriptors comprises, for at least one descriptor of said descriptors, employing an entropy decoding that is different from an entropy decoding used for at least one other descriptor of said descriptors.

17. An encoding apparatus comprising encoding means for carrying out the encoding method of any of claims 1 to 10.

18. A decoding apparatus comprising decoding means for carrying out the decoding method of any of claims 11 to 16.

19. Storage means storing a computer program comprising instructions for executing the methods of any of claims 1 to 16.

**Patentansprüche**

1. Computerimplementiertes Verfahren zum Codieren von Genomsequenz-Daten, wobei die Genomsequenz-Daten Reads von Sequenzen von Nukleotiden umfassen, wobei das Verfahren die Schritte umfasst:

Alignieren der Reads mit einer oder mehreren Referenzsequenzen, wodurch alignierte Reads geschaffen werden,
Klassifizieren der alignierten Reads in unterschiedliche Klassen gemäß angegebenen Übereinstimmungsregeln mit der einen oder den mehreren Referenzsequenzen, wobei das Klassifizieren umfasst:

- Klassifizieren eines oder mehrerer der alignierten Reads in eine erste Klasse (Klasse P), wenn gefunden wird, dass eine Region in der Referenzsequenz ohne jeglichen Übereinstimmungsfehler mit dem alignierten Read übereinstimmt;
- Klassifizieren eines oder mehrerer der alignierten Reads in eine zweite Klasse (Klasse N), wenn gefunden wird, dass eine Region in der Referenzsequenz mit dem alignierten Read mit einem Typ und einer Anzahl von Übereinstimmungsfehlern übereinstimmt, die nur durch die Anzahl von Positionen bestimmt wird, in welcher eine Sequenziermaschine, welche das Read erzeugt hat, nicht in der Lage war, eine Base zu nennen;
- Klassifizieren eines oder mehrerer der alignierten Reads in eine dritte Klasse (Klasse M), wenn gefunden wird, dass eine Region in der Referenzsequenz mit dem alignierten Read mit einem Typ und einer Anzahl von Übereinstimmungsfehlern übereinstimmt, die nur durch die Anzahl von Positionen bestimmt wird, in welcher die Sequenziermaschine, welche das Read erzeugt hat, nicht in der Lage war, eine Base zu nennen; plus die Anzahl von Übereinstimmungsfehlern, in welchen ein unterschiedliches Nukleotid als jenes, das in der Referenzsequenz vorliegt, genannt wurde;
- Klassifizieren eines oder mehrerer der alignierten Reads in eine vierte Klasse (Klasse I), die durch alignierte Reads gebildet wird, die zumindest einen Übereinstimmungsfehler eines beliebigen Typs aus Insertion, Deletion und Clipping plus, sofern vorhanden, jegliche Übereinstimmungsfehlertypen, die zu der zweiten Klasse (Klasse N) oder der dritten Klasse (Klasse M) gehören, aufweisen, wobei Insertionen durch eine zusätzliche Sequenz eines oder mehrerer Nukleotide, die in der Referenzsequenz nicht vorhanden sind, aber in dem alignierten Read vorhanden sind, gebildet werden, wobei Deletionen in dem alignierten Read fehlende Nukleotide relativ zu der Referenzsequenz darstellen, und wobei das Clipping weich abgeschnittene Nukleotide umfasst, die eine inserierte Nukleotidsequenz an den Rändern des alignierten Reads darstellen, die nicht mit der Referenzsequenz übereinstimmen, jedoch in dem alignierten Read gehalten werden, sowie hart abgeschnittene Nukleotide, die aus dem alignierten Read verworfen werden;
- Klassifizieren eines oder mehrerer der alignierten Reads in eine fünfte Klasse (Klasse U), wenn keine Übereinstimmung gemäß einer der ersten bis vierten Klasse gefunden wird;

wodurch Klassen von alignierten Reads geschaffen werden; die durch Gruppen von genomischen Deskriptoren repräsentiert werden, die eindeutig Genomsequenz-Reads repräsentieren, und die in Blöcken mit homogenen statistischen Eigenschaften organisiert sind,
wobei die Deskriptoren für die erste Klasse (Klasse P) umfassen:

- die Mapping-Position eines Reads auf der Referenzsequenz (pos),
- den DNA- oder RNA-Strang, auf welchen der Read abgebildet wurde (rcomp), und
- Mapping-Flags, die es dem Aligner ermöglichen, das Ergebnis des Mapping-Prozesses näher zu spezifizieren;

wobei die Deskriptoren ferner für die zweite Klasse (Klasse N) umfassen:

- die Position von Übereinstimmungsfehlern in alignierten Reads in Bezug auf Referenzsequenzen (mmpos);

wobei die Deskriptoren ferner für die dritte Klasse (Klasse M) umfassen:

- die Typen von Übereinstimmungsfehlern in Bezug auf Referenzsequenzen an den zugehörigen Positionen (mmtype);

wobei die Deskriptoren ferner für die vierte Klasse (Klasse I) umfassen:

- einen Deskriptor, der weich oder hart abgeschnittene Nukleotide (Clips) signalisiert;

wobei die Deskriptoren ferner für die fünfte Klasse (Klasse U) umfassen:

- wortwörtliche Sequenz-Reads von Basen, die nicht auf irgendeine verfügbare Referenzsequenz abgebildet werden können (ureads);

Codieren der klassifizierten alignierten Reads, die durch Blöcke von Deskriptoren mit homogenen statistischen Eigenschaften repräsentiert werden, als eine Vielzahl von Blöcken von Syntaxelementen, und
Strukturieren der Blöcke von Syntaxelementen mit Header-Informationen, wodurch aufeinander folgende Zugriffseinheiten geschaffen werden,
wobei das Codieren ferner das Binarisieren und Entropie-Codieren der genomischen Deskriptoren umfasst,
wobei das Binarisieren und Entropie-Codieren in Übereinstimmung mit den jeweiligen deskriptor-spezifischen statistischen Eigenschaften durchgeführt wird,
wobei das Binarisieren und Entropie-Codieren von genomischen Deskriptoren so erfolgt, dass die Binarisierung für zumindest einen Deskriptor der Deskriptoren sich von der Binarisierung für zumindest einen weiteren Deskriptor der Deskriptoren unterscheidet.

2. Verfahren nach Anspruch 1, ferner umfassend das Codieren eines oder mehrerer der folgenden Deskriptoren:

Deskriptor rlen zum Signalisieren der Länge eines jeden codierten Sequenz-Reads;
mmap zum Signalisieren der mehreren Mapping-Positionen, die einem einzelnen Read oder einem Paar von Reads durch den Mapping-Vorgang zugeordnet werden;
msar zum Signalisieren der Identifikation der Existenz von gespleißten Reads (d.h., Reads, die, wenn sie in Chunks geteilt werden, Mapping-Positionen mit höherer Übereinstimmungsgenauigkeit finden, als wenn sie als ein einzelner durchgehender Read abgebildet werden, der auf eine einzelne Position auf einer Referenzsequenz abgebildet wird).
Deskriptor mscore zum Signalisieren einer Mapping/Ausrichtungs-Einstufung je Read, wie er durch Genomsequenz-Read-Aligner erzeugt wird;

Deskriptor pair zum Signalisieren, im Fall von verpaarten End-Reads, wie die Reads verpaart sind;

Deskriptor rtype, der verwendet wird, um die Teilmenge von Deskriptoren zu signalisieren, die verwendet werden, um Sequenz-Reads zu codieren, die nicht mit angegebenen Graden der Übereinstimmungsgenauigkeit auf irgendeine Position der Referenzsequenz abgebildet werden können;

Deskriptor rgroup zum Signalisieren, zu welcher Read-Gruppe der Read gehört;

rftp zum Signalisieren der Position von Übereinstimmungsfehlern zwischen einem Contig und einer Referenzsequenz, wobei Positionen von Übereinstimmungsfehlern durch ein spezielles Terminator-Zeichen abgeschlossen werden,

rftt zum Signalisieren des Typs von Übereinstimmungsfehlern zwischen einem Contig und einer Referenzsequenz.

3. Verfahren nach Anspruch 1 oder 2, wobei die Deskriptoren wie folgt binarisiert werden:

der Deskriptor pos wird unter Verwendung eines doppelt abgeschnittenen unären Codes oder eines einfachen doppelt abgeschnittenen unären Codes binarisiert;

der Deskriptor rcomp wird unter Verwendung eines abgeschnittenen unären Codes binarisiert;

die Mapping-Flags werden unter Verwendung der binären Codierung binarisiert;

der Deskriptor mmpos zum Signalisieren der Position von Übereinstimmungsfehlern in alignierten Reads in Bezug auf Referenzsequenzen wird unter Verwendung eines geteilten, einheitsweise abgeschnittenen unären Codes binarisiert;

der Deskriptor mmtype zum Signalisieren der Typen von Übereinstimmungsfehlern in Bezug auf Referenzsequenzen an den zugeordneten Positionen wird unter Verwendung eines abgeschnittenen unären Codes binarisiert;

der Deskriptor clips zum Signalisieren von weich oder hart abgeschnittenen Nukleotiden wird unter Verwendung einer Verkettung von vorzeichenbehafteten abgeschnittenen Exponential-Golomb-, abgeschnittenen unären, vorzeichenbehafteten Exponential-Golomb- und binären Codes binarisiert.

4. Verfahren nach Anspruch 2, oder Anspruch 3 sofern abhängig von Anspruch 2, wobei einer oder mehrere der Deskriptoren wie folgt binarisiert werden:

der Deskriptor rlen, der die Länge eines jeden codierten Sequenz-Reads signalisiert, wird unter Verwendung eines geteilten, einheitsweise abgeschnittenen unären Codes binarisiert;

der Deskriptor mmap zum Signalisieren der mehreren Mapping-Positionen, die einem einzelnen Read oder einem Paar von Reads durch den Mapping-Vorgang zugeordnet werden, wird unter Verwendung eines geteilten, einheitsweise abgeschnittenen unären Codes binarisiert;

der Deskriptor msar zum Signalisieren der Identifikation der Existenz von gespleißten Reads wird unter Verwendung eines Signed-Exponential-Golomb-Codes binarisiert;

der Deskriptor mscore zum Signalisieren einer Mapping/Ausrichtungs-Einstufung je Read, wie er durch Genomsequenz-Read-Aligner erzeugt wird, wird unter Verwendung eines abgeschnittenen unären Codes binarisiert;

der Deskriptor pair zum Signalisieren, im Fall von verpaarten End-Reads, wie die Reads verpaart sind, wird unter Verwendung einer Verkettung von binärer Codierung und geteiltem, einheitsweise abgeschnittenen unären Code binarisiert;

der Deskriptor ureads zum Signalisieren von Reads, die nicht an irgendeiner Position auf der Referenzsequenz aligniert werden konnten, wird unter Verwendung eines abgeschnittenen unären Codes binarisiert;

der Deskriptor rtype, der verwendet wird, um die Teilmenge von Deskriptoren zu signalisieren, die verwendet werden, um Sequenz-Reads zu codieren, die nicht mit angegebenen Graden der Übereinstimmungsgenauigkeit auf irgendeine Position der Referenzsequenz abgebildet werden können, wird unter Verwendung eines abgeschnittenen unären Codes binarisiert;

der Deskriptor rgroup zum Signalisieren, zu welcher Read-Gruppe der Read gehört, wird unter Verwendung eines abgeschnittenen unären Codes binarisiert;

der Deskriptor rftp zum Signalisieren der Position von Übereinstimmungsfehlern zwischen einem Contig und einer Referenzsequenz wird unter Verwendung einer Verkettung von binärer Codierung und geteiltem, einheitsweise abgeschnittenem unären Code binarisiert; und

der Deskriptor rftt zum Signalisieren des Typs von Übereinstimmungsfehlern zwischen einem Contig und einer Referenzsequenz wird unter Verwendung einer Verkettung von binärer Codierung und abgeschnittenem unären Code binarisiert.

**5.** Verfahren nach einem der vorhergehenden Ansprüche, wobei die Deskriptoren durch Einfügen von Konfigurationsparametern in einen Syntax-Header codiert werden.

**6.** Verfahren nach Anspruch 5, wobei die Konfigurationsparameter aktualisiert werden, indem aktualiesierte Syntax-Header erzeugt werden, die zu der codierten genomischen Datei hinzugefügt werden.

**7.** Verfahren nach Anspruch 5 oder 6, wobei die Konfigurationsparameter einen oder mehrere der folgenden umfassen:

ein Datensatz-Typ zur Angabe der Art der in den Zugriffseinheiten codierten Daten, die auf diese Konfigurationsparameter Bezug nehmen;
eine Read-Länge zum Signalisieren der Länge der Sequenz-Reads in Nukleotiden im Falle einer konstanten Read-Länge;
einen Parameter für die Tiefe der Qualitätswerte zum Signalisieren der Anzahl der Qualitätswerte, die jedem codierten Nukleotid zugeordnet sind;
eine Alignment-Einstufungstiefe, die die Anzahl der Alignment-Einstufungswerte signalisiert, die jedem codierten Alignment zugeordnet sind;
eine Terminator-Größe zum Signalisieren der Größe des Terminator-Symbols, das für den Deskriptor mmpos verwendet wird, in Bytes;
einen Terminator-Wert zum Signalisieren des Werts des Terminator-Symbols, das für den Deskriptor mmpos verwendet wird;
die Anzahl von Klassen zum Signalisieren der Anzahl von Datenklassen, die in allen Zugriffseinheiten codiert sind, die auf die Konfigurationsparameter Bezug nehmen;
Klassen-Identifikatoren zum Signalisieren der jeder Datenklasse zugeordneten Identifikatoren;
die Anzahl der Deskriptoren zum Signalisieren der Gesamtzahl der in den Zugriffseinheiten enthaltenen Deskriptoren, die auf die Konfigurationsparameter Bezug nehmen;
Codiermodus-Identifikatoren für die unterschiedlichen Codier-Modi;
eine Anzahl von Gruppenparametern zum Signalisieren der Anzahl von unterschiedlichen Werten des Deskriptors rgroup, die in allen Zugriffseinheiten vorhanden sind, die auf die aktuellen Konfigurationsparameter Bezug nehmen;
einen oder mehrere Gruppennamen-Parameter zum Signalisieren eines oder mehrere Read-Gruppen-Identifikatoren;
ein Mehrfach-Alignment-Flag zum Signalisieren des Vorliegens von mehreren Alignments in der Zugriffseinheit; und
ein Gespleißte-Reads-Flag zum Signalisieren des Vorliegens von gespleißten Reads in der Zugriffseinheit.

**8.** Verfahren nach einem der Ansprüche 5 bis 7, wobei die Konfigurationsparameter ferner ein Mehrfach-Signatur-Basisflag zum Signalisieren der Verwendung mehrerer Signaturen in einer Zugriffseinheit umfassen, die nicht abgebildete Sequenz-Reads (Klasse U) enthält, wobei vorzugsweise die Konfigurationsparameter ferner eine Signaturgröße zum Signalisieren der Größe einer jeden ganzen Zahl, die eine codierte Signatur darstellt, in Bits umfassen.

**9.** Verfahren nach einem der vorhergehenden Ansprüche, wobei das Entropie-Codieren der Deskriptoren unter Verwendung einer Mehrzahl von Entropie-Codierern durchgeführt wird. I

**10.** Verfahren nach Anspruch 9, wobei die Binarisierung und Entropie-Codierung derart erfolgt, dass die Entropie-Codierung für zumindest einen Deskriptor der Deskriptoren sich von der Entropie-Codierung für zumindest einen weiteren Deskriptor der Deskriptoren unterscheidet.

**11.** Computerimplementiertes Verfahren zum Decodieren von codierten Genomsequenz-Daten, wobei die Genomsequenz-Daten Reads von Sequenzen von Nukleotiden umfassen, wobei das Verfahren die Schritte umfasst:

Parsen von Zugriffseinheiten, die codierte genomische Daten enthalten, zum Extrahieren von mehreren Blöcken von genomischen Deskriptoren unter Verwendung von Header-Informationen; und
Decodieren der Vielzahl von Blöcken;
wobei das Decodieren der Vielzahl von Blöcken das Entropie-Decodieren und Debinarisieren von Deskriptoren umfasst, die in Blöcken von homogenen Daten angeordnet sind, um eine Extraktion von aliginierten Reads gemäß spezifischen Übereinstimmungsregeln, welche deren Klassifikation in Bezug auf eine oder mehrere Referenzsequenzen definieren, auszuführen,
wobei:

- wenn einer oder mehrere der alignierten Reads in eine erste Klasse (Klasse P) klassifiziert sind, die Extraktion auf Grundlage einer Region in der Referenzsequenz ausgeführt wird, die mit dem einen oder den mehreren alignierten Reads ohne jeglichen Übereinstimmungsfehler übereinstimmt;

- wenn einer oder mehrere der alignierten Reads in eine zweite Klasse (Klasse N) klassifiziert sind, die Extraktion auf Grundlage einer Region in der Referenzsequenz ausgeführt wird, die mit dem einen oder den mehreren alignierten Reads mit einem Typ und einer Anzahl von Übereinstimmungsfehlern übereinstimmt, die nur durch die Anzahl von Positionen bestimmt wird, in welcher eine Sequenziermaschine, welche das Read erzeugt hat, nicht in der Lage war, eine Base zu nennen;

- wenn einer oder mehrere der alignierten Reads in eine dritte Klasse (Klasse M) klassifiziert ist, die Extraktion auf Grundlage einer Region in der Referenzsequenz ausgeführt wird, die mit dem einen oder den mehreren alignierten Reads mit einem Typ und einer Anzahl von Übereinstimmungsfehlern übereinstimmt, die nur durch die Anzahl von Positionen bestimmt wird, in welcher die Sequenziermaschine, welche das Read erzeugt hat, nicht in der Lage war, eine Base zu nennen; plus die Anzahl von Übereinstimmungsfehlern, in welchen ein unterschiedliches Nukleotid als jenes, das in der Referenzsequenz vorliegt, genannt wurde;

- wenn einer oder mehrere der alignierten Reads in eine vierte Klasse (Klasse I) klassifiziert ist, die Extraktion auf Grundlage des einen oder der mehreren alignierten Reads ausgeführt wird, die zumindest einen Übereinstimmungsfehler eines beliebigen Typs aus Insertion, Deletion und Clipping plus, sofern vorhanden, jegliche Übereinstimmungsfehlertypen, die zu der zweiten Klasse (Klasse N) oder der dritten Klasse (Klasse M) gehören, aufweisen, wobei Insertionen durch eine zusätzliche Sequenz eines oder mehrerer Nukleotide, die in der Referenzsequenz nicht vorhanden sind, aber in dem alignierten Read vorhanden sind, gebildet werden, wobei Deletionen in dem alignierten Read fehlende Nukleotide relativ zu der Referenzsequenz darstellen, und wobei das Clipping weich abgeschnittene Nukleotide umfasst, die eine inserierte Nukleotidsequenz an den Rändern des alignierten Reads darstellen, die nicht mit der Referenzsequenz übereinstimmen, jedoch in dem alignierten Read gehalten werden, sowie hart abgeschnittene Nukleotide, die aus dem alignierten Read verworfen werden;

- wenn einer oder mehrere der alignierten Lesungen in eine fünfte Klasse (Klasse U) klassifiziert sind, die Extraktion auf Grundlage der Tatsache erfolgt, dass keine Übereinstimmung gemäß einer der ersten bis vierten Klasse vorliegt;

wobei die Deskriptoren für die erste Klasse (Klasse P) umfassen:

- die Mapping-Position eines Reads auf der Referenzsequenz (pos),
- den DNA- oder RNA-Strang, auf welchen der Read abgebildet wurde (rcomp), und
- Mapping-Flags, die es dem Aligner ermöglichen, das Ergebnis des Mapping-Prozesses näher zu spezifizieren;

wobei die Deskriptoren ferner für die zweite Klasse (Klasse N) umfassen:

- die Position von Übereinstimmungsfehlern in alignierten Reads in Bezug auf Referenzsequenzen (mmpos);

wobei die Deskriptoren ferner für die dritte Klasse (Klasse M) umfassen:

- die Typen von Übereinstimmungsfehlern in Bezug auf Referenzsequenzen an den zugehörigen Positionen (mmtype);

wobei die Deskriptoren ferner für die vierte Klasse (Klasse I) umfassen:

- einen Deskriptor, der weich oder hart abgeschnittene Nukleotide (Clips) signalisiert;

wobei die Deskriptoren ferner für die fünfte Klasse (Klasse U) umfassen:

- wortwörtliche Sequenz-Reads von Basen, die nicht auf irgendeine verfügbare Referenzsequenz abgebildet werden können (ureads);

wobei die Entropie-Decodierung und Debinarisierung für zumindest einen Deskriptor der Deskriptoren eine Debinarisierung einsetzt, die sich von einer Debinarisierung unterscheidet, die für zumindest einen weiteren Deskriptor der Deskriptoren verwendet wird.

12. Verfahren nach Anspruch 11, ferner umfassend das Decodieren des einen oder mehreren der folgenden Deskriptoren:

Deskriptor rlen zum Signalisieren der Länge eines jeden codierten Sequenz-Reads;

mmap zum Signalisieren der mehreren Mapping-Positionen, die einem einzelnen Read oder einem Paar von Reads durch den Mapping-Vorgang zugeordnet werden;

msar zum Signalisieren der Identifikation der Existenz von gespleißten Reads, d.h., Reads, die, wenn sie in Chunks geteilt werden, Mapping-Positionen mit höherer Übereinstimmungsgenauigkeit finden, als wenn sie als ein einzelner durchgehender Read abgebildet werden, der auf eine einzelne Position auf einer Referenzsequenz abgebildet wird;

Deskriptor mscore zum Signalisieren einer Mapping/Ausrichtungs-Einstufung je Read, wie er durch Genomsequenz-Read-Aligner erzeugt wird;

Deskriptor pair zum Signalisieren, im Fall von verpaarten End-Reads, wie die Reads verpaart sind;

Deskriptor ureads zum Signalisieren von Reads, die nicht an irgendeiner Position auf der Referenzsequenz aligniert werden konnten;

Deskriptor rtype, der verwendet wird, um die Teilmenge von Deskriptoren zu signalisieren, die verwendet werden, um Sequenz-Reads zu codieren, die nicht mit angegebenen Graden der Übereinstimmungsgenauigkeit auf irgendeine Position der Referenzsequenz abgebildet werden können;

Deskriptor rgroup zum Signalisieren, zu welcher Read-Gruppe der Read gehört;

rftp zum Signalisieren der Position von Übereinstimmungsfehlern zwischen einem Contig und einer Referenzsequenz, wobei Positionen von Übereinstimmungsfehlern durch ein spezielles Terminator-Zeichen abgeschlossen werden;

rftt zum Signalisieren des Typs von Übereinstimmungsfehlern zwischen einem Contig und einer Referenzsequenz.

13. Verfahren nach Anspruch 11 oder 12, wobei die Deskriptoren durch Extrahieren von Konfigurationsparametern aus einem Syntax-Header decodiert werden.

14. Verfahren nach Anspruch 13, wobei die Konfigurationsparameter einen oder mehrere der folgenden umfassen:

ein Datensatz-Typ zur Angabe der Art der in den Zugriffseinheiten codierten Daten, die auf diese Konfigurationsparameter Bezug nehmen;

eine Read-Länge m Signalisieren der Länge der Sequenz-Reads in Nukleotiden im Falle einer konstanten Read-Länge;

einen Parameter für die Tiefe der Qualitätswerte zum Signalisieren der Anzahl der Qualitätswerte, die jedem codierten Nukleotid zugeordnet sind;

eine Alignment-Einstufungstiefe, die die Anzahl der Alignment-Einstufungswerte signalisiert, die jedem codierten Alignment zugeordnet sind;

eine Terminator-Größe zum Signalisieren der Größe des Terminator-Symbols, das für den Deskriptor mmpos verwendet wird, in Bytes;

einen Terminator-Wert zum Signalisieren des Werts des Terminator-Symbols, das für den Deskriptor mmpos verwendet wird;

die Anzahl von Klassen zum Signalisieren der Anzahl von Datenklassen, die in allen Zugriffseinheiten codiert sind, die auf die Konfigurationsparameter Bezug nehmen;

Klassen-Identifikatoren zum Signalisieren der jeder Datenklasse zugeordneten Identifikatoren;

die Anzahl der Deskriptoren zum Signalisieren der Gesamtzahl der in den Zugriffseinheiten enthaltenen Deskriptoren, die auf die Konfigurationsparameter Bezug nehmen;

Codiermodus-Identifikatoren zum Signalisieren der unterschiedlichen Codier-Modi, die in dieser Offenbarung definiert sind;

eine Anzahl von Gruppenparametern zum Signalisieren der Anzahl von unterschiedlichen Werten des Deskriptors rgroup, die in allen Zugriffseinheiten vorhanden sind, die auf die aktuellen Konfigurationsparameter Bezug nehmen;

einen oder mehrere Gruppennamen-Parameter zum Signalisieren eines oder mehrere Read-Gruppen-Identifikatoren;

ein Mehrfach-Alignment-Flag zum Signalisieren des Vorliegens von mehreren Alignments in der Zugriffseinheit; und

ein Gespleißte-Reads-Flag zum Signalisieren des Vorliegens von gespleißten Reads in der Zugriffseinheit, mit der Maßgabe, dass keine gespleißten Reads vorliegen, wenn es auf 0 gesetzt ist.

**15.** Verfahren nach Anspruch 13, oder Anspruch 14, sofern abhängig von Anspruch 13, wobei die Konfigurationsparameter ferner ein Mehrfach-Signatur-Basisflag zum Signalisieren der Verwendung mehrerer Signaturen in einer Zugriffseinheit umfassen, die nicht abgebildete Sequenz-Reads (Klasse U) enthält; und die Konfigurationsparameter ferner vorzugsweise eine Signaturgröße zum Signalisieren der Größe einer jeden ganzen Zahl, die eine codierte Signatur darstellt, in Bits umfassen.

**16.** Verfahren nach einem der Ansprüche 11 bis 15, wobei die Entropie-Decodierung und Debinarisierung genomischer Deskriptoren für zumindest einen Deskriptor der Deskriptoren eine Entropie-Decodierung einsetzt, die sich von einer Entropie-Decodierung unterscheidet, die für zumindest einen weiteren Deskriptor der Deskriptoren verwendet wird.

**17.** Codiervorrichtung, umfassend Codiermittel zum Ausführen des Codierverfahrens nach einem der Ansprüche 1 bis 10.

**18.** Decodiervorrichtung, umfassend Decodiermittel zum Ausführen des Decodierverfahrens nach einem der Ansprüche 11 bis 16.

**19.** Speichermittel, die ein Computerprogramm speichern, das Anweisungen zum Ausführen der Verfahren nach einem der Ansprüche 1 bis 16 umfasst.

**Revendications**

**1.** Procédé mis en oeuvre par ordinateur pour encoder des données de séquence de génome, lesdites données de séquence de génome comprenant des lectures de séquences de nucléotides, ledit procédé comprenant les étapes consistant à :

aligner lesdites lectures sur une ou plusieurs séquences de référence, créant ainsi des lectures alignées, classifier lesdites lectures alignées en différentes classes conformément à des règles de correspondance spécifiées avec lesdites une ou plusieurs séquences de référence, dans lequel ladite étape de classification comprend :

- classifier une ou plusieurs desdites lectures alignées en une première classe (classe P) lorsqu'une région dans la séquence de référence, qui correspond à la lecture alignée sans aucun défaut de correspondance, est trouvée ;
- classifier une ou plusieurs desdites lectures alignées en une deuxième classe (classe N) lorsqu'une région dans la séquence de référence, qui correspond à la lecture alignée avec un type et un nombre de défauts de correspondance déterminés seulement par le nombre de positions dans lesquelles une machine de séquençage générant la lecture n'a été capable d'appeler aucune base, est trouvée ;
- classifier une ou plusieurs desdites lectures alignées en une troisième classe (classe M) lorsqu'une région dans la séquence de référence, qui correspond à la lecture alignée avec un type et un nombre de défauts de correspondance déterminés seulement par le nombre de positions dans lesquelles la machine de séquençage générant la lecture n'a été capable d'appeler aucune base, plus le nombre de défauts de correspondance dans lesquels un nucléotide différent de celui présent dans la séquence de référence a été appelé, est trouvée ;
- classifier une ou plusieurs desdites lectures alignées en une quatrième classe (classe I) constituée de lectures alignées présentant au moins un défaut de correspondance d'un quelconque type parmi insertion, suppression et écrêtés plus, s'il est présent, un quelconque type de défauts de correspondance appartenant à la deuxième classe (classe N) ou à la troisième classe (classe M), dans lequel les insertions sont constituées d'une séquence supplémentaire d'un ou plusieurs nucléotides non présents dans la séquence de référence, mais présents dans la lecture alignée, dans lequel les suppressions constituent des nucléotides manquants dans la lecture alignée par rapport à la séquence de référence, et dans lequel écrêtés comprend des nucléotides à écrêtage doux, qui représentent une séquence de nucléotides insérée sur les bords de la lecture alignée qui ne correspondent pas à la séquence de référence mais sont gardés dans la lecture alignée, et des nucléotides à écrêtage fort, qui sont écartés de la lecture alignée ;
- classifier une ou plusieurs desdites lectures alignées en une cinquième classe (classe U) lorsqu'aucune correspondance conformément à l'une quelconque de la première à la quatrième classe n'est trouvée ;

créer ainsi des classes de lectures alignées, représentées par des groupes de descripteurs génomiques qui représentent de manière univoque des lectures de séquence de génome et qui sont organisés en blocs avec des propriétés statistiques homogènes,
dans lequel les descripteurs comprennent, pour la première classe (classe P) :

- la position de cartographie d'une lecture sur la séquence de référence (pos),
- le brin d'ADN ou d'ARN sur lequel la lecture a été cartographiée (rcomp), et
- des drapeaux de cartographie pour permettre à l'aligneur de spécifier davantage le résultat du procédé de cartographie ;

dans lequel les descripteurs comprennent en outre, pour la deuxième classe (classe N) :

- la position de défauts de correspondance dans des lectures alignées par rapport à des séquences de référence (mmpos) ;

dans lequel les descripteurs comprennent en outre, pour la troisième (classe M) :

- les types de défauts de correspondance par rapport à des séquences de référence aux positions associées (mmtype) ;

dans lequel les descripteurs comprennent outre, pour la quatrième classe (classe I) :

- un descripteur signalant des nucléotides à écrêtage doux ou fort (clips) ;

dans lequel les descripteurs comprennent en outre, pour la cinquième classe (classe U) :

- des lectures de séquence intégrales de bases qui ne peuvent être cartographiées sur aucune séquence de référence disponible (ureads) ;

encoder lesdites lectures alignées classifiées représentées par des blocs de descripteurs avec des propriétés statistiques homogènes comme une multiplicité de blocs d'éléments syntaxiques, et
structurer lesdits blocs d'éléments syntaxiques avec des informations d'en-tête, créant ainsi des unités d'accès successives,
dans lequel ledit encodage comprend en outre la binarisation et le codage entropique desdits descripteurs génomiques, ladite binarisation et ledit codage entropique étant réalisés conformément à des propriétés statistiques spécifiques de chaque descripteur, dans lequel ladite binarisation et ledit codage entropique de descripteurs génomiques sont tels que la binarisation pour au moins un descripteur parmi lesdits descripteurs est différente de la binarisation pour au moins un autre descripteur parmi lesdits descripteurs.

2. Procédé selon la revendication 1, comprenant en outre le codage d'un ou plusieurs des descripteurs suivants :

un descripteur rien pour signaler la longueur de chaque lecture de séquence encodée ;
mmap pour signaler les multiples positions de cartographie qui sont associées à une unique lecture ou une paire de lectures par la procédure de cartographie ;
msar pour signaler l'identification de l'existence de lectures épissées (c'est-à-dire de lectures qui, lorsqu'elles sont divisées en fragments, trouvent des positions de cartographie avec de plus hauts degrés de précision de correspondance que lorsqu'elles sont cartographiées comme une unique lecture contiguë cartographiée sur une unique position sur une séquence de référence) ;
un descripteur mscore pour signaler un score de cartographie/alignement par lecture tel que généré par des aligneurs de lectures de séquence génomique ;
un descripteur pair pour signaler, en cas de lectures d'extrémité appariées, comment les lectures sont appariées ;
un descripteur rtype utilisé pour signaler le sous-ensemble de descripteurs utilisé pour encoder des lectures de séquence qui ne peuvent être cartographiées à aucune position de la séquence de référence avec des degrés spécifiés de précision de correspondance ;
un descripteur rgroup pour signaler à quel groupe de lectures la lecture appartient ;
rftp pour signaler la position de défauts de correspondance entre un contig et une séquence de référence, dans lequel des positions de défauts de correspondance sont terminées par un caractère de terminaison spécial ;
rftt pour signaler le type de défauts de correspondance entre un contig et une séquence de référence.

3. Procédé selon la revendication 1 ou 2, dans lequel lesdits descripteurs sont binarisés comme suit :

ledit descripteur pos est binarisé en utilisant un code unaire tronqué double ou un unique code unaire tronqué double ;

ledit descripteur rcomp est binarisé en utilisant un code unaire tronqué ;

lesdits descripteurs de drapeaux de cartographie sont binarisés en utilisant un codage binaire ;

ledit descripteur mmpos pour signaler la position de défauts de correspondance dans des lectures alignées par rapport à des séquences de référence est binarisé en utilisant un codage unaire tronqué dans le sens de l'unité divisée ;

ledit descripteur mmtype pour signaler les types de défauts de correspondance par rapport à des séquences de référence aux positions associées est binarisé en utilisant un code unaire tronqué ;

ledit descripteur clips pour signaler des nucléotides à écrêtage doux ou fort est binarisé en utilisant une concaténation de codes exponentiel-Golomb tronqué signé, unaire tronqué, exponentiel-Golomb signé et binaire.

4. Procédé selon la revendication 2 ou la revendication 3 lorsqu'elle dépend de la revendication 2, dans lequel un ou plusieurs desdits descripteurs sont binarisés comme suit :

ledit descripteur rien signalant la longueur de chaque lecture de séquence encodée est binarisé en utilisant un code unaire tronqué dans le sens de l'unité divisée ;

ledit descripteur mmap pour signaler les multiples positions de cartographie qui sont associées à une unique lecture ou une paire de lectures par la procédure de cartographie est binarisé en utilisant un code unaire tronqué dans le sens de l'unité divisée ;

ledit descripteur msar pour signaler l'identification de l'existence de lectures épissées est binarisé en utilisant un code exponentiel-Golomb signé ;

ledit descripteur mscore pour signaler un score de cartographie/alignement par lecture tel que généré par des aligneurs de lectures de séquence génomique est binarisé en utilisant un code unaire tronqué ;

ledit descripteur pair pour signaler, en cas de lectures d'extrémité appariées, comment les lectures sont appariées est binarisé en utilisant une concaténation de codage binaire et code unaire tronqué dans le sens de l'unité divisée ;

ledit descripteur ureads pour signaler des lectures qui n'ont pu être alignées à aucune position de la séquence de référence est binarisé en utilisant un code unaire tronqué ;

ledit descripteur rtype utilisé pour signaler le sous-ensemble de descripteurs utilisé pour encoder des lectures de séquence qui ne peuvent être cartographiées à aucune position de la séquence de référence avec des degrés spécifiés de précision de correspondance est binarisé en utilisant un code unaire tronqué ;

ledit descripteur rgroup pour signaler à quel groupe de lectures la lecture appartient est binarisé en utilisant un code unaire tronqué ;

ledit descripteur rftp pour signaler la position de défauts de correspondance entre un contig et une séquence de référence est binarisé en utilisant une concaténation de codage binaire et code unaire tronqué dans le sens de l'unité divisée ; et

ledit descripteur rftt pour signaler le type de défauts de correspondance entre un contig et une séquence de référence est binarisé en utilisant une concaténation de codage binaire et code unaire tronqué.

5. Procédé selon l'une quelconque des revendications précédentes, dans lequel lesdits descripteurs sont codés en insérant des paramètres de configuration dans un en-tête syntaxique.

6. Procédé selon la revendication 5, dans lequel lesdits paramètres de configuration sont mis à jour en créant des en-têtes syntaxiques mis à jour devant être ajoutés au fichier génomique encodé.

7. Procédé selon la revendication 5 ou 6, dans lequel lesdits paramètres de configuration comprennent un ou plusieurs de ce qui suit :

un type de jeu de données pour signaler le type de données encodées dans des unités d'accès faisant référence à ces paramètres de configuration ;

une longueur de lecture pour signaler la longueur en nucléotides de lectures de séquence en cas de longueur de lecture constante ;

un paramètre de profondeur de valeurs de qualité pour signaler le nombre de valeurs de qualité associées à chaque nucléotide codé ;

une profondeur de score d'alignement pour signaler le nombre de scores d'alignements associés à chaque alignement codé ;

une taille de terminaison pour signaler la taille en octets du symbole de terminaison utilisé pour le descripteur mmpos ;

une valeur de terminaison pour signaler la valeur du symbole de terminaison utilisé pour le descripteur mmpos ;

le nombre de classes pour signaler le nombre de classes de données encodées dans toutes les unités d'accès faisant référence auxdits paramètres de configuration ;

des identificateurs de classe pour signaler les identificateurs associés à chaque classe de données ;

le nombre de descripteurs pour signaler le nombre total de descripteurs contenus dans des unités d'accès faisant référence auxdits paramètres de configuration ;

des identificateurs de mode de codage pour les différents modes de codage ;

un paramètre de nombre de groupes pour signaler le nombre de valeurs différentes du descripteur rgroup présent dans toutes les unités d'accès faisant référence aux paramètres de configuration courants ;

un ou plusieurs paramètres de nom de groupe pour signaler un ou plusieurs identificateurs de groupe de lectures ;

un drapeau de multiples alignements pour signaler la présence de multiples alignements dans l'unité d'accès ; et

un drapeau de lectures épissées pour signaler la présence de lectures épissées dans l'unité d'accès.

**8.** Procédé selon l'une quelconque des revendications 5 à 7, dans lequel lesdits paramètres de configuration comprennent en outre un drapeau de base de multiples signatures pour signaler l'utilisation de multiples signatures dans une unité d'accès contenant des lectures de séquence non cartographiées (classe U), de préférence dans lequel lesdits paramètres de configuration comprennent en outre une taille de signature pour signaler la taille en bits de chaque entier représentant une signature encodée.

**9.** Procédé selon l'une quelconque des revendications précédentes, dans lequel ledit codage entropique desdits descripteurs est réalisé en utilisant une multiplicité de codeurs entropiques.

**10.** Procédé selon la revendication 9, dans lequel ladite binarisation et ledit codage entropique sont tels que le codage entropique pour au moins un descripteur parmi lesdits descripteurs est différent du codage entropique pour au moins un autre descripteur parmi lesdits descripteurs.

**11.** Procédé mis en oeuvre par ordinateur pour décoder des données génomiques codées, dans lequel lesdites données de séquence de génome comprennent des lectures de séquences de nucléotides, ledit procédé comprenant les étapes consistant à :

analyser des unités d'accès contenant lesdites données génomiques encodées pour extraire de multiples blocs de descripteurs génomiques en employant des informations d'en-tête ; et

décoder ladite multiplicité de blocs ;

dans lequel ledit décodage d'une multiplicité de blocs comprend le décodage entropique et la dé-binarisation de descripteurs génomiques agencés en blocs de données homogènes pour effectuer une extraction de lectures alignées conformément à des règles de correspondance spécifiques définissant leur classification par rapport à une ou plusieurs séquences de référence,

dans lequel :

- lorsqu'une ou plusieurs desdites lectures alignées sont classifiées en une première classe (classe P), ladite extraction est effectuée sur la base d'une région dans la séquence de référence correspondant aux une ou plusieurs lectures alignées sans aucun défaut de correspondance ;

- lorsqu'une ou plusieurs desdites lectures alignées sont classifiées en une deuxième classe (classe N), ladite extraction est effectuée sur la base d'une région dans la séquence de référence correspondant aux une ou plusieurs lectures alignées avec un type et un nombre de défauts de correspondance déterminés seulement par le nombre de positions dans lesquelles une machine de séquençage générant la lecture n'a été capable d'appeler aucune base ;

- lorsqu'une ou plusieurs desdites lectures alignées sont classifiées en une troisième classe (classe M), ladite extraction est effectuée sur la base d'une région dans la séquence de référence correspondant aux une ou plusieurs lectures alignées avec un type et un nombre de défauts de correspondance déterminés seulement par le nombre de positions dans lesquelles la machine de séquençage générant la lecture n'a été capable d'appeler aucune base, plus le nombre de défauts de correspondance dans lesquels un nucléotide différent de celui présent dans la séquence de référence a été appelé ;

- lorsqu'une ou plusieurs desdites lectures alignées sont classifiées en une quatrième classe (classe I), ladite extraction est effectuée sur la base desdites une ou plusieurs lectures alignées présentant au moins un défaut de correspondance d'un quelconque type parmi insertion, suppression et écrêtés plus, s'il est présent, un quelconque type de défauts de correspondance appartenant à la deuxième classe (classe N) ou à la troisième classe (classe M), dans lequel les insertions sont constituées d'une séquence supplémentaire d'un ou plusieurs nucléotides non présents dans la séquence de référence, mais présents dans la lecture alignée, dans lequel les suppressions constituent des nucléotides manquants dans la lecture alignée par rapport à la séquence de référence, et dans lequel écrêtés comprend des nucléotides à écrêtage doux, qui représentent une séquence de nucléotides insérée sur les bords de la lecture alignée qui ne correspondent pas à la séquence de référence mais sont gardés dans la lecture alignée, et des nucléotides à écrêtage fort, qui sont écartés de la lecture alignée ;

- lorsqu'une ou plusieurs desdites lectures alignées sont classifiées en une cinquième classe (classe U), ladite extraction est effectuée sur la base du fait qu'aucune correspondance conformément à l'une quelconque de la première à la quatrième classe n'est présente ;

dans lequel les descripteurs comprennent, pour la première classe (classe P) :

- la position de cartographie d'une lecture sur la séquence de référence (pos),
- le brin d'ADN ou d'ARN sur lequel la lecture a été cartographiée (rcomp), et
- des drapeaux de cartographie pour permettre à l'aligneur de spécifier davantage le résultat du procédé de cartographie ;

dans lequel les descripteurs comprennent en outre, pour la deuxième classe (classe N) :

- la position de défauts de correspondance dans des lectures alignées par rapport à des séquences de référence (mmpos) ;

dans lequel les descripteurs comprennent en outre, pour la troisième (classe M) :

- les types de défauts de correspondance par rapport à des séquences de référence aux positions associées (mmtype) ;

dans lequel les descripteurs comprennent outre, pour la quatrième classe (classe I) :

- un descripteur signalant des nucléotides à écrêtage doux ou fort (clips) ;

dans lequel les descripteurs comprennent en outre, pour la cinquième classe (classe U) :

- des lectures de séquence intégrales de bases qui ne peuvent être cartographiées sur aucune séquence de référence disponible (ureads) ;

dans lequel ledit décodage entropique et ladite dé-binarisation de descripteurs génomiques comprennent, pour au moins un descripteur parmi lesdits descripteurs, l'emploi d'une dé-binarisation qui est différente d'une dé-binarisation utilisée pour au moins un autre descripteur parmi lesdits descripteurs.

**12.** Procédé selon la revendication 11, comprenant en outre le décodage des un ou plusieurs parmi les descripteurs suivants :

un descripteur rien pour signaler la longueur de chaque lecture de séquence encodée ;
mmap pour signaler les multiples positions de cartographie qui sont associées à une unique lecture ou une paire de lectures par la procédure de cartographie ;
msar pour signaler l'identification de l'existence de lectures épissées, c'est-à-dire de lectures qui, lorsqu'elles sont divisées en fragments, trouvent des positions de cartographie avec de plus hauts degrés de précision de correspondance que lorsqu'elles sont cartographiées comme une unique lecture contiguë cartographiée sur une unique position sur une séquence de référence ;
un descripteur mscore pour signaler un score de cartographie/alignement par lecture tel que généré par des aligneurs de lectures de séquence génomique ;
un descripteur pair pour signaler, en cas de lectures d'extrémité appariées, comment les lectures sont appariées ;
un descripteur ureads pour signaler des lectures qui n'ont pu être alignées sur aucune position de la séquence de référence ;

un descripteur rtype utilisé pour signaler le sous-ensemble de descripteurs utilisé pour encoder des lectures de séquence qui ne peuvent être cartographiées à aucune position de la séquence de référence avec des degrés spécifiés de précision de correspondance ;

un descripteur rgroup pour signaler à quel groupe de lectures la lecture appartient ;

rftp pour signaler la position de défauts de correspondance entre un contig et une séquence de référence, dans lequel des positions de défauts de correspondance sont terminées par un caractère de terminaison spécial ;

rftt pour signaler le type de défauts de correspondance entre un contig et une séquence de référence.

13. Procédé selon la revendication 11 ou 12, dans lequel lesdits descripteurs sont décodés en extrayant des paramètres de configuration d'un en-tête syntaxique.

14. Procédé selon la revendication 13, dans lequel lesdits paramètres de configuration comprennent un ou plusieurs de ce qui suit :

un type de jeu de données pour signaler le type de données encodées dans des unités d'accès faisant référence à ces paramètres de configuration ;

une longueur de lecture pour signaler la longueur en nucléotides de lectures de séquence en cas de longueur de lecture constante ;

un paramètre de profondeur de valeurs de qualité pour signaler le nombre de valeurs de qualité associées à chaque nucléotide codé ;

une profondeur de score d'alignement pour signaler le nombre de scores d'alignements associés à chaque alignement codé ;

une taille de terminaison pour signaler la taille en octets du symbole de terminaison utilisé pour le descripteur mmpos ; une valeur de terminaison pour signaler la valeur du symbole de terminaison utilisé pour le descripteur mmpos ;

le nombre de classes pour signaler le nombre de classes de données encodées dans toutes les unités d'accès faisant référence auxdits paramètres de configuration ;

des identificateurs de classe pour signaler les identificateurs associés à chaque classe de données ;

le nombre de descripteurs pour signaler le nombre total de descripteurs contenus dans des unités d'accès faisant référence auxdits paramètres de configuration ;

des identificateurs de mode de codage pour signaler les modes de codage définis dans cette description ;

un paramètre de nombre de groupes pour signaler le nombre de valeurs différentes du descripteur rgroup présent dans toutes les unités d'accès faisant référence aux paramètres de configuration courants ;

un ou plusieurs paramètres de nom de groupe pour signaler un ou plusieurs identificateurs de groupe de lectures ;

un drapeau de multiples alignements pour signaler la présence de multiples alignements dans l'unité d'accès ; et

un drapeau de lectures épissées pour signaler la présence de lectures épissées dans l'unité d'accès, à condition que lorsqu'il est positionné sur 0, aucune lecture épissée ne soit présente.

15. Procédé selon la revendication 13 ou la revendication 14 lorsqu'elle dépend de la revendication 13, dans lequel lesdits paramètres de configuration comprennent en outre un drapeau de base de multiples signatures pour signaler l'utilisation de multiples signatures dans une unité d'accès contenant des lectures de séquence non cartographiées (classe U) ; et de préférence lesdits paramètres de configuration comprennent en outre une taille de signature pour signaler la taille en bits de chaque entier représentant une signature encodée.

16. Procédé selon l'une quelconque des revendications 11 à 15, dans lequel ledit décodage entropique et ladite débinarisation de descripteurs génomiques comprennent, pour au moins un descripteur parmi lesdits descripteurs, l'emploi d'un décodage entropique qui est différent d'un décodage entropique utilisé pour au moins un autre descripteur parmi lesdits descripteurs.

17. Appareil d'encodage comprenant des moyens d'encodage pour effectuer le procédé d'encodage selon l'une quelconque des revendications 1 à 10.

18. Appareil de décodage comprenant des moyens de décodage pour effectuer le procédé de décodage selon l'une quelconque des revendications 11 à 16.

19. Moyens de stockage stockant un programme d'ordinateur comprenant des instructions pour exécuter les procédés selon l'une quelconque des revendications 1 à 16.

Coordinate system on a
«Reference Sequence»

Figure 1.

$\Delta i$ = i<sup>th</sup> genomic interval on the reference sequence

AU$i$ = i<sup>th</sup> Access Unit encapsulating compressed descriptors representing sequences mapped in interval $\Delta i$

**Figure 2.**

Figure 3.

Figure 4.

Figure 5.

Figure 6

Figure 7

Figure 8.

Left-most read has N alignments    The right-most read has $P = \sum_{i=1}^{N} M_i$ alignments

Reference

Alignments of the right-most read associated with the first alignment of the left-most read

Alignments of the right-most associated with the $N^{th}$ alignment of the left-most read

**Figure 9.**

**Figure 10.**

Alignments of the right -most read
associated with the alignment
of the left-most read

...

Alignments of the right -most read
associated with the $N^{th}$ mapping position of
the left-most read

Figure 11.

pos stream

pair stream

mmap stream

msar stream

N = total number of pos descriptors for this record
NP = total number of pair descriptors for this record
N+P = total number of msar descriptors for this record

**Figure 12.**

**Figure 13.**

Figure 14.

Figure 15.

Figure 16.

X = mismatch that will disappear

Y = mismatch that will not disappear but mismatch code shall change

W = mismatch that stays unchanged

Z = Newly introduced mismatch after transformation

Figure 17.

Reference sequence 1

Figure 18.

Class P encoded reads $\boxed{P_0 \mid \ldots \mid P_K}$ $\xrightarrow{\text{Encoded using } R_0}$ $\boxed{P_0 \mid \ldots \mid P_K}$

Class N encoded reads $\boxed{N_0 \mid \ldots \mid N_K}$ $\boxed{T(N_0) \mid \ldots \mid T(N_K)}$

Class M encoded reads $\boxed{M_0 \mid \ldots \mid M_K}$ $\xrightarrow{\text{Encoded using } R_1}$ $\boxed{T(M_0) \mid \ldots \mid T(M_K)}$

Class I encoded reads $\boxed{I_0 \mid \ldots \mid I_K}$ $\quad 1900 \boxed{R_1 = A_0(R_0)}$ $\boxed{T(I_0) \mid \ldots \mid T(I_K)}$

T represents the transformation of each encoded read when encoded with respect to $R_1 = A_0(R_0)$

Class P encoded reads $\boxed{P_0 \mid \ldots \mid P_K}$ $\xrightarrow{\text{Encoded using } R_0}$ $\boxed{P_0 \mid \ldots \mid P_K}$

Class N encoded reads $\boxed{N_0 \mid \ldots \mid N_K}$ $1901 \boxed{R_N = A_N(R_0)}$ $\boxed{T_N(N_0) \mid \ldots \mid T_N(N_K)}$

Class M encoded reads $\boxed{M_0 \mid \ldots \mid M_K}$ $1902 \boxed{R_M = A_M(R_0)}$ $\boxed{T_M(M_0) \mid \ldots \mid T_M(M_K)}$

Class I encoded reads $\boxed{I_0 \mid \ldots \mid I_K}$ $1903 \boxed{R_I = A_I(R_0)}$ $\boxed{T_I(I_0) \mid \ldots \mid T_I(I_K)}$

Encoded using $R_N R_M R_I$

**Figure 19.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CRAM format specification (version 3.0),* 08 September 2016, https://samtools.github.io/hts-specs/CRAMv3.pdf

- Comparison of high-throughput sequencing data compression tools. **NUMANAGIC, I. et al.** Nature Methods. Nature Publishing Group, 2016, vol. 13, 1005-1008